# EUROPEAN PATENT APPLICATION

(11) **EP 4 759 925 A1**
(43) Date of publication of application: **17.06.2026**
(21) Application number: 24219517.0
(22) Date of filing: 12.12.2024
(51) Int. Cl.: C12N 5/0797, C12N 5/079, C12N 5/09, C12N 5/071

(54) **METHOD FOR GENERATING A TUMOR ASSEMBLOID**

(71) Applicant: Eberhard Karls Universität Tübingen (Medizinische Fakultät), 72074 Tübingen (DE)
(72) Inventor: Arnold, Vanessa, 71126 Gäufelden-Nebringen (DE); Wolfram, Anna, 72076 Tübingen (DE); Sevenich, Lisa, 72127 Kusterdingen (DE)
(74) Representative: Witte, Weller & Partner Patentanwälte mbB

(57) **Abstract**

The invention relates to a method for generating a tumor assembloid, to a tumor assembloid obtainable by this method, to a method for generating a tumor spheroid assembloid, and to a tumor spheroid assembloid obtainable by said method.

## Description

### FIELD OF THE INVENTION

The invention relates to a method for generating a tumor assembloid, to a tumor assembloid obtainable by this method, to a method for generating a tumor spheroid assembloid, and to a tumor spheroid assembloid obtainable by said method.

### BACKGROUND OF THE INVENTION

The treatment of primary and secondary brain cancers continues to present significant challenges despite advanced multimodal regimens. The median survival is still only 10 months. The imperative for novel treatments is thus clear. Yet, the current drug development landscape sees only a 7% success rate. This is largely due to the disconnect between *in vitro* and *in vivo* models, particularly in assessing neurotoxicity and tumor microenvironment dynamics.

Presently, the iterative drug design process is significantly hindered by the poor translation of results from controlled *in vitro* environments to complex *in vivo* conditions. Conventional *in vitro* models are often inadequate in evaluating factors such as neurotoxicity and the intricate interactions within the tumor microenvironment. Additionally, initial *in vivo* screenings involving extensive drug libraries are facing increasing ethical restrictions coupled with a steady increase in costs. As a matter of fact, the European Union has launched a major program for the reduction, replacement, and refinement (3Rs) in the use of animals for scientific research.

To address these challenges, significant strides have been made in creating models compliant with the 3Rs, such as *in vitro* spheroids - three-dimensional (3D) cancer cell aggregates - and *ex vivo* organoids - 3D complex clusters of organ-specific cells - from animal and human tissues. These models, particularly cerebral organoids, have been instrumental in mirroring brain development and pathology, as they can reproduce *in vivo*-like 3D environments. Typically, these organoids are cultivated from induced pluripotent (iPSCs) or embryonic stem cells (eSCs).

Prior art document WO 2023/073006 describes the development of a model system called 'midbrain assembloids'. This system combines three-dimensional organ-like cell cultures with multiple cell types to potentially better model the function of the human midbrain. The focus is particularly on the integration of microglia into midbrain organoids to study their neuroinflammatory and synaptic functions.

WO 2024/123942 discloses methods for creating chimeric organoids, which are three-dimensional structures containing cells from different origins, including multiple individuals, brain regions, species, or embryological sources. These organoids aim to enhance the understanding of human brain development, neurophysiological processes, and inter-individual genetic variability in response to diseases or treatments.

EP 4 410 962 describes methods and compositions for producing cell aggregates containing pituitary hormone-producing cells derived from pluripotent stem cells, such as induced pluripotent stem cells (iPSCs) or embryonic stem cells (eSCs). The authors aim to address challenges in efficiently generating functional pituitary cells for therapeutic and research applications.

However, these methods known in the art have important drawbacks. They are resource-intensive and prone to variability in cell composition across batches. Furthermore, these well-known models often do not realistically and consistently reproduce the tumor architecture and single models display great batch variation. Invasiveness and metastasis are also not well demonstrated in the known models and methods. This limits their utility in systematic screenings essential for drug discovery.

### SUMMARY

In view of this, the object of the present invention is to avoid or at least reduce the disadvantages described above in the prior art. In particular, a method is to be provided for producing a cellular construct that realistically and consistently reflects the tumor architecture and thus enables the discovery of promising anti-tumor drugs. This should help to overcome the 'reproducibility crisis' in research, as well as the lack of technical repeatability and the associated comparability of data between models and species, particularly in preclinical research.

The object underlying the invention is solved by a method of generating a tumor assembloid comprising the steps of:
(1) providing a single organoid cell suspension;
(2) providing a single tumor cell suspension;
(3) mixing cells of the single organoid cell suspension with cells of the single tumor cell suspension in a predetermined ratio to obtain an organoid cell-tumor cell mixture;
(4) culturing the organoid cell-tumor cell mixture to obtain the tumor assembloid.

The inventors are the first to provide a tumor assembloid model that not only mimics the clinical tumor architecture but reproducibly exhibits 'predefined' composition of distinct cell populations and thus enables realistic and consistent tumor studies. The method according to the invention delivers, as an end product, a three-dimensional assembloid with a 'core' of tumor cells that is completely surrounded by healthy cells of an organoid. The use of precisely predetermined cells from both single-cell suspensions makes the method and the resulting tumor assembloid highly reproducible. Thanks to the invention, the tumor assembloid can be formed very quickly, e.g. within 72 hours.

According to the invention, an 'assembloid' is an *in vitro* generated three-dimensional cell structure that is created by combining at least two different cell types or organoids to mimic the cellular architecture and function of a complex tissue or organ system. In a 'tumor assembloid' according to the invention, one cell type is a tumor cell and another cell type is a cell of (an) organoid(s).

According to the invention, an 'organoid' is a three-dimensional cell structure grown *in vitro* that typically develops from stem cells or progenitor-like cells and replicates key structural and functional properties of a specific organ (e.g. brain) or tissue in the body.

According to the invention, a 'single organoid cell suspension' as provided in step (1) is a suspension of isolated cells that have been detached from each other and that originate from an organoid. It should be noted that in the 'single organoid cell suspension', the cells do not necessarily have to originate from a single organoid, although they may, but may also originate from several organoids.

A 'single tumor cell suspension' as provided in step (2) is a suspension of isolated cells that have been detached from another and originate from one or more tumors or one or more tumor cell lines, respectively.

For the purpose of singling or isolation of the cells, measures known to those skilled in the art are taken, such as trypsinization, centrifugation, washing, neutralization and resuspension, etc.

In mixing step (3), aliquots of both cell types are brought in contact and/or incubated together to obtain a co-culture of cells from the organoid(s) and cells from the tumor(s) or tumor cell line(s).

The ratio of the two cell types is 'predetermined'. This means that a precisely defined number of single cells from the organoid(s) is mixed or incubated with a precisely defined number of single cells from the tumor(s) or tumor cell line(s).

Cultivation in step (4) is carried out under conditions known in the art to obtain an assembloid and is based on a range of conditions the skilled person is aware of, such as the type of tumor and organoid cells, the targeted size of the assembloid, etc. For example, in one embodiment, cultivation may include an initial growing phase without agitation followed by a cultivation phase with agitation. In further embodiments, temperatures of 37°C and/or gassing with CO₂ may be provided.

The inventors have surprisingly discovered that by precisely adjusting the number of single tumor and organoid cells to be mixed, a tumor assembloid can be created in which a core of tumor cells is completely surrounded by healthy cells of the organoid. Because of this architecture this structure is herein referred as to the 'cancer core' or 'cancer core model'. This cancer core structure allows for an improved exemplification and study of cell-cell interaction between healthy and pathogenic cell populations as well as the infiltrative and/or metastatic properties of malignant tumors, as well as the targeted identification of therapeutic agents with specific focus on simultaneous efficacy and neurotoxicity evaluation in the context of drug screening.

These properties are particularly valuable for the investigation and, potentially, future treatment of tumors, such as brain tumors. Current state-of-the-art models are not able to adequately represent the cell-cell interaction in brain tumor microenvironment. This is a critical shortcoming, since tumor cell-brain cell communication strongly impacts tumor progression and treatment outcome. Further, state-of-the-art systems mainly focus on primary brain cancer, which severely limits research into these incurable tumor types. In the known models, the cancer cells mainly grow on the surface of the cerebral organoids, which restricts the physiological relevance of these models and leads to results that are difficult to transfer into clinical practice.

The current, known models are time-consuming and therefore not efficient enough for effective drug screening, which delays the development of new therapeutic approaches.

The invention provides a solution for the first time. It bridges the gap between existing human *ex vivo* and murine *in vivo* models to close the translation gap and significantly improve the precision of preclinical studies.

Particularly noteworthy is that the tumor assembloid according to the invention can be generated within a short time (e.g., 72 hours), is highly reproducible, and has little batch effects. This makes it ideal for a variety of applications, including drug screening, tumor invasion studies, and combination studies. The tumor assembloid shows characteristic features of the disease, depending on the type of cancer and cell composition, which gives it a high clinical relevance.

In another embodiment of the method according the invention, the predetermined ratio of the number of cells of the single organoid cell suspension to the number of cells of the single tumor cell suspension is in the range of approx. 1:1 to approx. 10,000:1.

This measure has the advantage that the skilled person obtains a specific range of the number of cells to be used of each cell type. The inventors have found that, with cell number ratios that lie within the specified range, particularly good tumor assembloids according to the invention are obtained. Approx. 1:1 to approx. 1:10,000 means that, for 1 organoid cell, all cell number values for the tumor cells are included that lie between 1 and 10,000, i.e., for example, approximately 1, 2, 3, 4, 5, 6, 7, 8, 9, 10, ..., 100, ..., 200, ..., 300, ..., 400, ..., 500, ..., 1,000, ..., 2,000, ..., 3,000, ..., 4,000, ..., 5,000, ..., 6,000, ..., 7,000, ..., 8,000, ..., 9,000, etc. The three dots (...) stand for all whole numbers between the preceding and following numerical value.

Here and in the context of the entire disclosure, "approximately" or "approx." means that usual fluctuations due to technical or measurement-related reasons are acceptable and included. It is understood, and is clear to the person skilled in the art, that an adjustment accurate to an individual, single cell is hardly possible, nor necessary to achieve the advantages of the invention. Slight deviations, which are covered by the term "approximately" or "approx.", are therefore within the scope of the invention.

In an embodiment of the method according to the invention the predetermined ratio of the number of cells of the single organoid cell suspension to the number of cells of the single tumor cell suspension is in a range selected from the group consisting of: approx. 2:1, approx. 4:1, approx. 10:1, approx. 20:1, approx. 100:1, approx. 1,000:1, approx. 2,000:1, approx. 5,000:1, approx. 10,000:1.

This measure has the advantage that the cell number ratios are further refined and, according to the inventors, lead to particularly good tumor assembloids. Depending on the chosen cancer cells or cell lines and cell ratios, the inventors observed tumor assembloids with well-defined cancer cores and/or with highly invasive, diffused cancer entities throughout the organoid. As a rule of thumb, 1:100 to 5:100 cancerous cells to non-cancerous organoid cells typically results in infiltrative patterns, while ratios from 1:10 usually exhibits more solid tumor cores.

In another embodiment of the invention the tumor cells are derived from a solid tumor, preferably from a tumor of human origin, further preferably from a solid tumor of human origin.

The use of tumor cells derived from solid tumors ensures great clinical relevance, since solid tumors represent one of the most common forms of cancer in humans. This embodiment further enables the use of tumor cells from human sources, which supports the development of patient-specific models for personalized medicine. It goes without saying that the tumor cells are preferably derived from a (solid) tumor of human origin, but not exclusively therefrom.

In still another embodiment of the invention the tumor cells are selected from the group consisting of: brain cancer cells, breast cancer cells, lung cancer cells, skin cancer cells.

This embodiment has the advantage of broadly covering a variety of clinically relevant cancer types. This enables the modeling of a broad range of tumor entities that commonly occur in patients. Each cancer type mentioned has specific biological properties (e.g. invasiveness, metastasis) that can be specifically modeled in the tumor assembloids according to the invention. The selection allows researchers to create models tailored to specific questions such as tumor microenvironment, immune interactions or drug testing. Examples of brain cancer cells include, but are not limited thereto, glioblastoma cells (GBM), a highly aggressive and infiltrative tumor of the central nervous system. Another example of brain cancer cells is astrocytoma cells, which are found in a slow-growing but potentially malignant brain tumor. Examples of breast cancer cells include HER2-positive breast cancer cells and triple-negative breast cancer cells. Examples of lung cancer cells include adenocarcinoma cells, which are the most common form of non-small cell lung cancer (NSCLC), and small cell lung cancer cells (SCLC), a highly aggressive tumor that metastasizes quickly. Examples of skin cancer cells include melanoma cells, which metastasize readily and are highly mutagenic, and basal cell carcinoma cells, which are found in the most common form of skin cancer with locally invasive growth.

In yet a further embodiment of the invention the tumor cells are selected from the group consisting of: glioma cells; breast-to-brain cancer cells; lung-to-brain cancer cells; melanoma-to-brain cancer cells.

This measure allows the method to be directed in a beneficial way towards tumor cells that either occur primarily in the brain (gliomas) or metastasize to the brain (e.g. breast, lung or melanoma cells). This enables a targeted investigation of brain tumors and their pathological properties. Brain metastases are among the most common and complex complications in cancer patients. The model according to the invention provides, therefore, a platform for investigating their biology and therapeutic target structures.

In still another embodiment of the invention the organoid cells are derived from a cerebral organoid.

Cerebral organoids mimic important components of the brain microenvironment, such as neuronal interactions and glial cell function. The use of cells from cerebral organoids enables a realistic replication of brain structure that surrounds the cancer core, which is particularly important for research into brain tumors or metastatic processes in the brain, as well as for the investigation of individual tumor developments and therapy options.

In a further embodiment of the method according to the invention the cerebral organoid was obtained from cells selected from the group consisting of: adult neural stem cells (aNSCs), embryonic stem cells (eSC), and induced pluripotent stem cells (iPSC).

This measure provides a convenient source of organoid cells. Adult neural stem cells (aNSCs) are multipotent stem cells found in the adult brain. They reside in specific niches, e.g. the subventricular zone and hippocampus. aNSCs can differentiate into neurons, astrocytes, and oligodendrocytes. They represent the natural brain microenvironment of adult tissues and allow the modeling of processes that occur in the mature brain, such as neurodegenerative diseases or tumor invasion. aNSCs are particularly useful for studying brain tumors (e.g., glioblastomas) and their interactions with mature neuronal structures. They provide a model for adult tumor processes, such as infiltration and interaction with astrocytic and oligodendrocytic cell types. Embryonic stem cells (eSCs) are pluripotent stem cells derived from the inner cell mass of a blastocyst. eSCs can differentiate into almost any cell type in the body. Their high level of differentiation allows the replication of various stages of brain development. eSCs-based organoids can model early tumor development in embryonic tissues. Induced pluripotent stem cells (iPSCs) are somatic cells that have been reprogrammed into a pluripotent state. iPSCs can be created from the cells of a specific patient, enabling personalized disease modeling. Unlike eSCs, they do not require an embryonic source, thus minimizing ethical concerns. iPSC-based organoids allow the study of patient-specific tumor processes, including individual genetic and epigenetic influences.

In another embodiment of the invention said cerebral organoid was grown for at least 1 month.

According to this embodiment of the invention, the organoid cells are obtained and singularized from such an organoid that has been cultivated for at least one month. This measure has the advantage of obtaining organoid cells that already show a sufficient degree of differentiation. It ensures that the organoid cells according to the invention create an environment for the cancer core that comes as close as possible to the pathological conditions in the patient. After one month of cultivation, organoids, such as cerebral organoids, develop more complex and mature structures, such as neuronal networks. These more closely mimic natural brain structures and create a more realistic environment for interaction with tumor cells. Prolonged cultivation promotes differentiation of stem cells into functional cell types such as neurons, astrocytes, and oligodendrocytes. Organoids that are cultured longer show more developed cell-cell contacts and synaptic interactions. This is crucial for studying how tumor cells interact with the brain microenvironment. Cells from mature organoids also respond more realistically to drugs, which improves the predictability of treatment outcomes. Cultivating cerebral organoids for at least 1 month prior to cell collection therefore offers decisive advantages for the maturation, functionality and biological relevance of the organoid cells. This optimizes the method, particularly with regard to modeling tumors that originate or metastasize in the brain, as well as for investigating innovative therapies.

In a further development of the invention the method comprises the following further steps:
(2') providing a single immune cell suspension;
(3') mixing cells of the single organoid cell suspension with cells of the single tumor cell suspension and with cells of the single immune cell suspension in a predetermined ratio to obtain an organoid cell-tumor cell-immune cell mixture, and
(4') culturing the organoid cell-tumor cell-immune cell mixture to obtain an 'advanced' tumor assembloid.

By this measure, the method according to the invention is further developed. It combines tumor cells with cerebral organoid cells and additionally integrates immune cells, such as microglia, to more comprehensively simulate the tumor microenvironment (TME). This model provides an accurate replication of clinically relevant tumor microenvironments, especially for high-grade gliomas or brain metastases such as breast cancer or melanoma metastases. By integrating immune cells, immunotherapies can be tested and their effect on tumor cells and brain tissue can be examined. This further developed method is herein referred as to the 'advanced cancer core' or 'advanced cancer core model'. The advanced cancer core method according to the invention enables simultaneous screening of therapeutic efficacy and neurotoxicity while also assessing the effects on the immune compartment and the influence of immune cell populations on therapy outcomes, thereby enhancing its translation into clinical applications.

According to the invention, a "single immune cell suspension" as provided in step (2') is a suspension of isolated immune cells, e.g. microglia cells, that are separated from each other.

In step (3'), aliquots of all three cell types are brought into contact and/or incubated together to obtain a co-culture of cells of the organoid(s), cells of the tumor(s) or tumor cell line(s), and immune cells.

The ratio of the three cell types is 'predetermined'. This means that a precisely defined number of single cells from the organoid(s) are mixed or incubated with a precisely defined number of single cells from the tumor(s) or tumor cell line(s) and a precisely defined number of immune cells.

Cultivation in step (4') is carried out under conditions known to those skilled in the art to obtain an organoid and is based on a range of conditions, such as the type of tumor and organoid cells, and the immune cells, the targeted size of the assembloid, etc. For example, in one embodiment, it may include an initial cultivation phase without agitation followed by a cultivation phase with agitation. In further embodiments, temperatures of 37°C and/or gassing with CO₂ may be provided.

In still another embodiment of the invention the predetermined ratio of the number of cells of the single organoid cell suspension to the number of cells of the single tumor cell suspension and to the number of cells of the single immune cell suspension is in a range of approx. 2:1:1 to approx. 100:1:100.

This measure has the advantage that the skilled person obtains a specific range of the number of cells to be used of each cell type relative to each other. The inventors have found that particularly good 'advanced' tumor assembloids according to the invention are obtained with cell number ratios that lie within the specified range. Approx. 2:1 to approx. 100:1 means that, for 1 tumor cell, all cell number values for the organoid cells are included that lie between 2 and 100, i.e., for example, approximately 2, 3, 4, 5, 6, 7, 8, 9, 10, 11, 12, 13, 14, 15, 16, 17, 18, 19, 20, ..., 30, ..., 40, ... 50, ..., 60, ..., 70, ..., 80, ..., 90, ..., 100. The three dots (...) stand for all whole numbers between the preceding and following numerical value.

In still another embodiment of the invention the immune cells are cells selected from the group consisting of: microglia cells, monocytes, macrophages, T lymphocytes, B lymphocytes, and dendritic cells.

By this measure specific immune cells are integrated into the method according to the invention, which are of particular relevance in brain tumor environment. The integration of these immune cell types enables a realistic simulation of the complex brain tumor microenvironment. In particular, immune reactions, inflammatory processes and the interaction between tumor cells and immune cells are simulated. This is crucial for the study of tumor immune evasion and immunomodulation. The presence of immune cells, such as microglia and macrophages, increases the complexity of the model, as these cells interact directly with tumor cells and cerebral organoid cells. Adaptive immune cells such as T and B lymphocytes increase the functional diversity of the system and allow the simulation of systemic immune responses. Immune cells in the assembloids allow the simultaneous evaluation of active substances with regard to their effectiveness against tumor cells and their effects on immune cells. This improves the predictability of how new drugs work in an immunologically active environment. By integrating patient-specific immune cells, personalized tumor models can be created. This enables the investigation of individual immune responses and targeted therapies.

In a further development of the method according to the invention the culturing in step (4) and optionally step (4') is carried out for approx. 72 hours and, optionally, at approx. 37°C and/or approx. 5% CO₂.

These precise conditions optimize the growth and interactions between the different cell types in the assembloid, including tumor cells, organoid cells, and optionally immune cells. Tumor cells exhibit realistic growth behavior under these conditions, including invasive and proliferative properties. Organoid cells retain their structure and functionality, enabling precise modeling of the tissue. The standardized conditions (time, temperature, CO₂) minimize variability between experiments and ensure consistent results. This facilitates the comparability of data, especially when conducting drug testing. The relatively short cultivation time of 72 hours enables rapid results, which are particularly valuable in the early phases of drug development. At the same time, the time is sufficient to establish complex structures and interactions within the assembloid.

In another embodiment of the invention the culturing in step (4) and optionally step (4') is carried out without shaking and, optionally, for approx. 24 hours, and/or optionally with shaking for approx. further 48 hours, preferably by using an orbital shaker. In still another embodiment after step (4) and optionally step (4'), in step (5) the culturing is carried out with shaking, optionally for approx. further 48 hours, and/or at approx. 37°C, and/or approx. 5% CO₂, preferably by using an orbital shaker.

The inventors have determined that the specified conditions are optimized for obtaining functional tumor assembloids. Cultivation at 37°C and 5% CO₂ mimics the optimal conditions for cell growth, proliferation, and differentiation. This is crucial for the formation of realistic tumor microenvironments. The consistent shaking of developing cancer core models increases the supply of nutrients and oxygen. The use of an orbital shaker ensures an even distribution of nutrients and oxygen in the culture. Compared to other shaking methods, an orbital shaker provides smooth agitation that preserves the integrity of the three-dimensional structure. This is particularly important for larger cell aggregates, as central cells can suffer from hypoxia in static conditions. An extended cultivation phase under shaking conditions allows complex cell interactions and gradients to establish themselves within the tumor assembloid. This reproduces the tumor architecture and microenvironment even more precisely.

Another subject-matter of the invention relates to the tumor assembloid obtainable by the 'cancer core' or 'advanced cancer core' method according to the invention.

The features, characteristics and advantages disclosed for the method according to the invention apply likewise to the tumor assembloid according to the invention.

Another subject-matter of the invention relates to a method of generating a tumor spheroid assembloid comprising the steps of:
(1) providing a single organoid cell suspension;
(2) providing preformed tumor spheroid(s);
(3) mixing cells of the single organoid cell suspension with the preformed tumor spheroid(s) in a predetermined ratio to obtain an organoid cell-tumor spheroid(s) mixture;
(4) culturing the organoid cell-tumor spheroid(s) mixture to obtain the tumor spheroid assembloid.

The features, characteristics, definitions and advantages described for the initial 'cancer core method' according to the invention apply to this method, referred to as 'spheroid core' or 'spheroid core model', in a likewise manner.

In this method, instead of 'single tumor cell suspension' so-called 'pre-formed tumor spheroids' are used. This measure increases cell density in the cancer compartment resulting in hypoxic or necrotic cores as seen in solid tumors in the clinic. Compared to the 'cancer core' method, this method offers the possibility of studying dense solid tumors, which allows a more realistic examination of hypoxia-associated resistance to treatments.

According to the invention 'preformed tumor spheroids' are three-dimensional cell cultures consisting of tumor cells grown *in vitro* under conditions that promote natural cell aggregation and self-assembly. These spheroids reflect important features of tumors, such as cell-cell interactions - tumor cells form tight contacts with each other, similar to a real tumor; gradient formation - oxygen and nutrient gradients, the central cells are limited in availability, which mimics hypoxia and resistance mechanisms; structural heterogeneity - outer cells are typically more proliferative, while central cells might be less active or necrotic. Spheroids are an ideal platform for evaluating the effect of drugs on complex tumor structures, including penetration and efficacy against central and peripheral tumor cells.

According to the invention, a 'tumor spheroid assembloid' is a three-dimensional cell culture model generated *in vitro* that is created by combining preformed tumor spheroids with cells from organoids (e.g. brain organoids), which surround the tumor spheroid. This model recapitulates key structural, functional and dynamic features of the tumor microenvironment (TME), including cell-cell interactions, oxygen and nutrient gradients as well as hypoxia-driven tumor heterogeneity.

In still another embodiment of said method the predetermined ratio of the number of cells of the single organoid cell suspension to the number of tumor spheroids is approx. 10,000 to approx. 500,000 cells to approx. a single preformed tumor spheroid.

This measure is used to determine the optimal ratio of the number of cells of the organoid to the number of spheroids based on the findings of the inventors. This ensures the consistency of the model and comparability between experiments. According to the invention, 10,000 to 500,000 includes all values in between, such as 10,000, ..., 20,000, ..., 30,000, ..., 40,000, ..., 50,000, ..., 60,000, ..., 70,000, ..., 80,000, ..., 90,000, ..., 100,000, ..., 200,000, ..., 300,000, ..., 400,000, ..., 500,000. The three dots (...) stand for all whole numbers between the preceding and following numerical value.

In yet another embodiment of the invention the single preformed tumor spheroid comprises approx. 1,000 to approx. 100,000 tumor cells.

A cell number of 1,000 to 100,000 tumor cells allows the formation of a spheroid that is sufficiently large to reproduce important tumor biological properties, such as cell-cell interactions or gradient formation. A spheroid with this cell number reaches a size similar to clinical tumor structures. It forms core regions with slow proliferating or necrotic cells as well as active, proliferating cell layers at the periphery. The cell number is sufficiently large to be applicable to various tumor models (e.g. highly proliferative or invasive tumors). At the same time, the size of the spheroid remains small enough to allow the diffusion of nutrients and drugs, making it ideal for drug testing. Spheroids in this size range can be effectively mixed with organoid cells without losing their three-dimensional structure. This improves integration into the tumor assembloid and enables realistic tumor microenvironment interactions. The specified cell number makes the production of spheroids reproducible and standardizable, which is crucial for scientific studies and preclinical applications. Tumor spheroids of this size are ideal for evaluating drug penetration and efficacy. They allow the investigation of how drugs affect central and peripheral cells in the spheroid. According to the invention, 1,000 to 100,000 includes all values in between, such as 1,000, ..., 2,000, ..., 3,000, ..., 4,000, ..., 5,000, ..., 6,000, ..., 7,000, ..., 8,000, ..., 9,000, ..., 10,000, ..., 20,000, ..., 30,000, ..., 40,000, ..., 50,000, ..., 60,000, ..., 70,000, ..., 80,000, ..., 90,000, ..., 100,000. The three dots (...) stand for all whole numbers between the preceding and following numerical value.

In a further embodiment of the invention said method comprises the following further steps:
(2') providing a single immune cell suspension;
(3') mixing cells of the single organoid cell suspension with the preformed tumor spheroids and with cells of the single immune cell suspension in a predetermined ratio to obtain an organoid cell-tumor spheroid-immune cell mixture, and
(4') culturing the organoid cell-tumor spheroid-immune cell mixture to obtain an 'advanced' tumor spheroid assembloid.

By this measure, the 'spheroid core' is further developed to form the 'advanced spheroid core" or 'advanced spheroid core model', respectively. It combines pre-formed tumor spheroids with organoid cells and additionally integrates immune cells, such as microglia, to more comprehensively simulate the tumor microenvironment (TME). This model provides an accurate replication of clinically relevant tumor microenvironments exhibiting hypoxia, especially for all solid cancer types prone to transient and/or static hypoxia. By integrating immune cells, therapy strategies including but not limited to immunotherapies can be tested and their effect on tumors and brain tissue can be examined. The 'advanced spheroid core' allows simultaneous screening of therapeutic efficacy and neurotoxicity while also assessing the effects on the immune compartment and theinfluence of immune cell populations on therapy outcomes, thereby improving its translation into clinical applications.

According to the invention, a 'single immune cell suspension' as provided in step (2') is a suspension of isolated immune cells, e.g. microglia cells, that are separated from each other.

In step (3'), aliquots of all three cell or structure types are brought into contact and/or incubated together to obtain a co-culture of cells from the organoid(s), the preformed tumor spheroid(s), and immune cells.

The ratio of the three cell types or structures is 'predetermined'. This means that a precisely defined number of single cells of the organoid(s) is mixed or incubated with a precisely defined number of preformed tumor spheroid(s) and a precisely defined number of immune cells.

Cultivation in step (4') is carried out under conditions known to those skilled in the art to obtain an 'advanced' tumor spheroid assembloid and is based on a range of conditions, such as the type of preformed tumor spheroid and organoid cells, and the immune cells, the targeted size of the assembloid, etc. For example, in one embodiment, it may include an initial cultivation phase without agitation followed by a cultivation phase with agitation. In further embodiments, temperatures of 37°C and/or gassing with CO₂ may be provided.

In still another embodiment of the invention, the predetermined ratio of the cells of the single organoid cell suspension to the number of preformed tumor spheroids and to the number of cells of the single immune cell suspension is approx. 10,000 to approx. 500,000 cells of the single organoid cell suspension to approx. a single preformed tumor spheroid and to approx. 10,000 to approx. 500,000 cells of the single immune cell suspension.

According to the invention, 10,000 to 500,000 includes all values in between, such as 10,000, ..., 20,000, ..., 30,000, ..., 40,000, ..., 50,000, ..., 60,000, ..., 70,000, ..., 80,000, ..., 90,000, ..., 100,000, ..., 200,000, ..., 300,000, ..., 400,000, ..., 500,000. The three dots (...) stand for all whole numbers between the preceding and following numerical value.

These cell numbers and ratios are precisely matched to ensure a realistic simulation of the tumor microenvironment. The ratio of approx. 10,000 to approx. 500,000 organoid cells to approx. one tumor spheroid ensures that the tumor spheroid is embedded in a sufficiently large environment that simulates natural interaction with healthy tissue. The integration of approx. 10,000 to approx. 500,000 immune cells complements the microenvironment by simulating immune reactions, inflammatory processes and tumor immune evasion.

The cell numbers create a balance between tumor cells, immune cells and healthy tissue cells. This enables the investigation of tumor invasion into healthy tissue, interactions between tumor and immune cells, and differentiated immune responses in the TME. The defined cell numbers and ratios improve the standardization of the model, making experiments reproducible and results more comparable. The specified cell numbers and tumor spheroid ratios are carefully balanced to closely mimic the complexity and functionality of the tumor microenvironment. They provide an ideal basis for investigating tumor growth, immune responses, and drug effects, while ensuring reproducibility and adaptability.

In another embodiment of said method the culturing in step (4) and optionally step (4') is carried out without shaking and, optionally, for approx. 24 hours, and/or at approx. 37°C and/or approx. 5% CO₂. Preferably, after step (4'), in step (4") the culturing is carried out with shaking, optionally for further approx. 48 hours, and/or at approx. 37°C, and/or approx. 5% CO₂, further preferably by using an orbital shaker.

The inventors have determined that the specified conditions are optimized for obtaining functional advanced tumor spheroid assembloids. During the first 24 hours without shaking, the formation of stable cell-cell contacts between the tumor, organoid, and optional immune cells is promoted. This enables uniform integration of the cell types and the formation of a consistent three-dimensional model. Sensitive cells, such as certain organoid or immune cells, benefit from a static environment that minimizes mechanical stress and improves cell viability. Static cultivation promotes initial adhesion and interaction of the different cell types. Cultivation at 37°C and 5% CO₂ mimics optimal conditions for cell growth, proliferation, and differentiation. This is crucial for the formation of realistic tumor microenvironments. The subsequent introduction of shaking increases the supply of nutrients and oxygen. The use of an orbital shaker ensures an even distribution of nutrients and oxygen in the culture. Compared to other shaking methods, an orbital shaker provides smooth agitation that preserves the integrity of the three-dimensional structure. An extended cultivation phase under shaking conditions allows complex cell interactions and gradients to establish themselves within the tumor assembloid. This reproduces the tumor architecture and microenvironment even more precisely.

Another subject-matter of the invention is a tumor spheroid assembloid obtainable by the afore-disclosed method according to the invention.

The features, characteristics, definitions and advantages described for the initial methods according to the invention apply to the tumor spheroid assembloid in a likewise manner.

The invention is now further explained by means of embodiments and examples resulting in additional features, characteristics and advantages of the invention. The embodiments and examples are of pure illustrative nature and do not limit the scope or range of the invention. The features mentioned in the specific embodiments and examples are features of the invention and may be seen as general features which are not applicable in the specific embodiment or example but also in an isolated manner in the context of any embodiment or example of the invention.

Reference is made to the following figures:

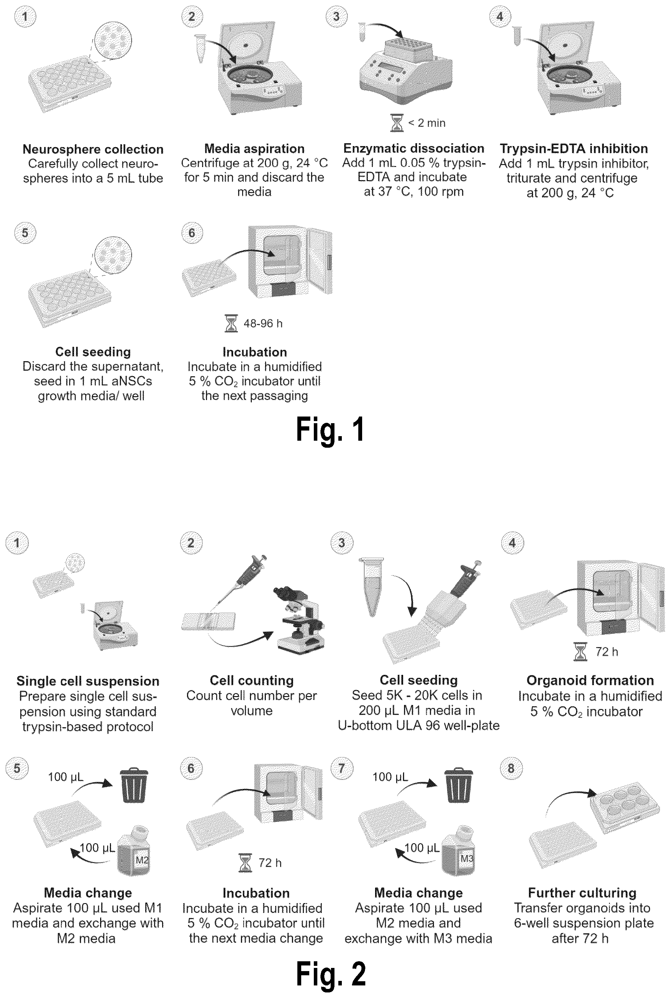

- Fig. 1:: Schematic Illustration of the aNSCs passaging workflow.
- Fig. 2:: Schematic illustration of cerebral organoid culture workflow.

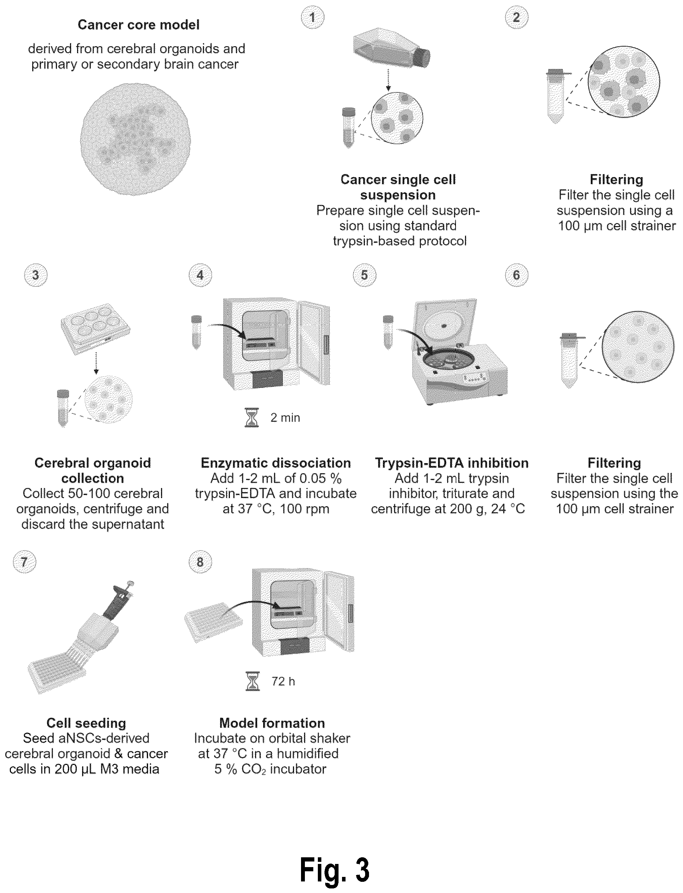

- Fig. 3:: Schematic illustration for cancer core model formation.

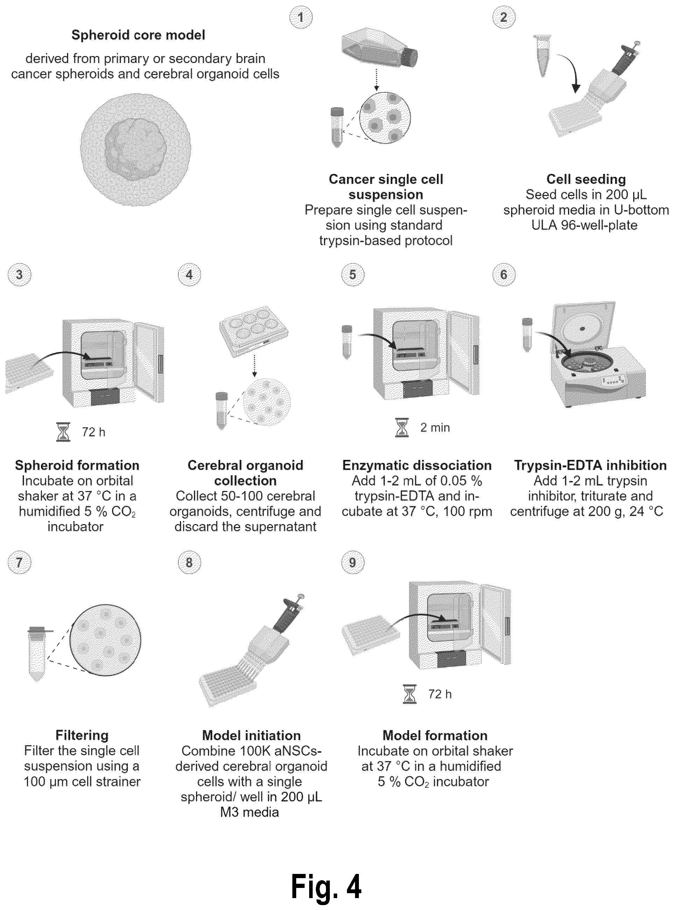

- Fig. 4:: Schematic illustration for spheroid core model formation.

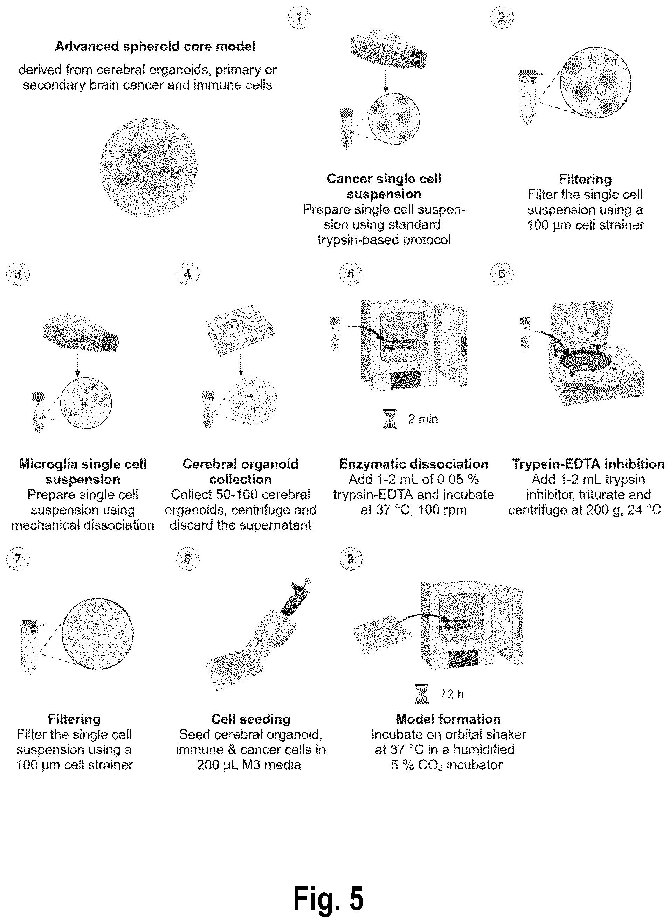

- Fig. 5:: Schematic illustration for advanced cancer core model formation.

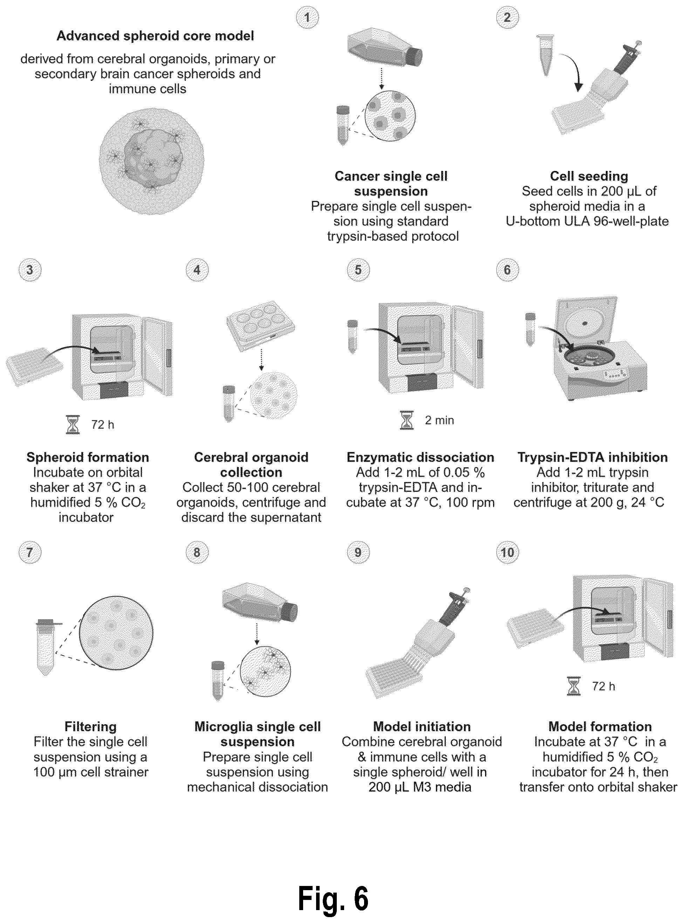

- Fig. 6:: Schematic illustration for advanced spheroid core model formation.

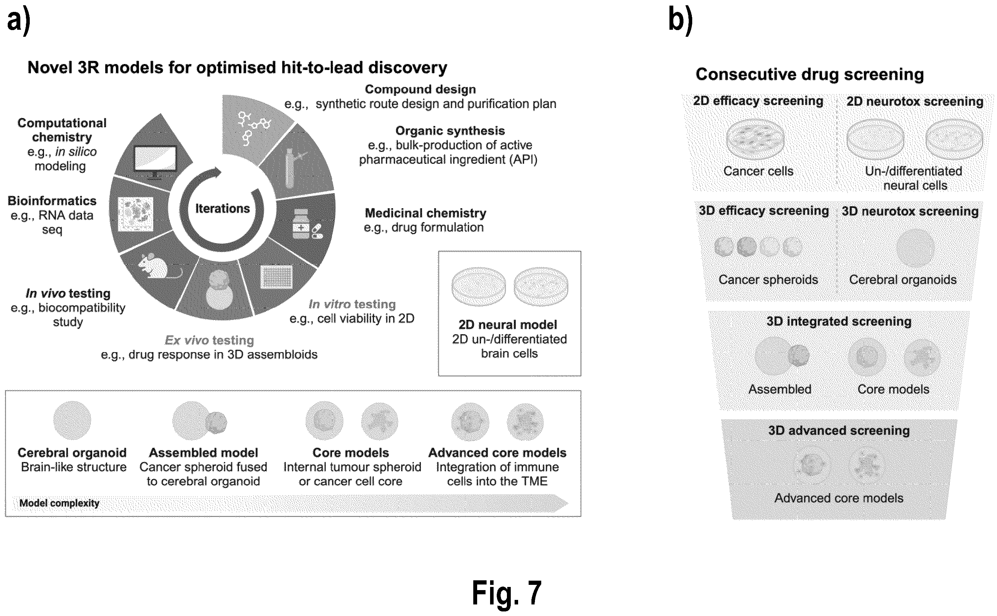

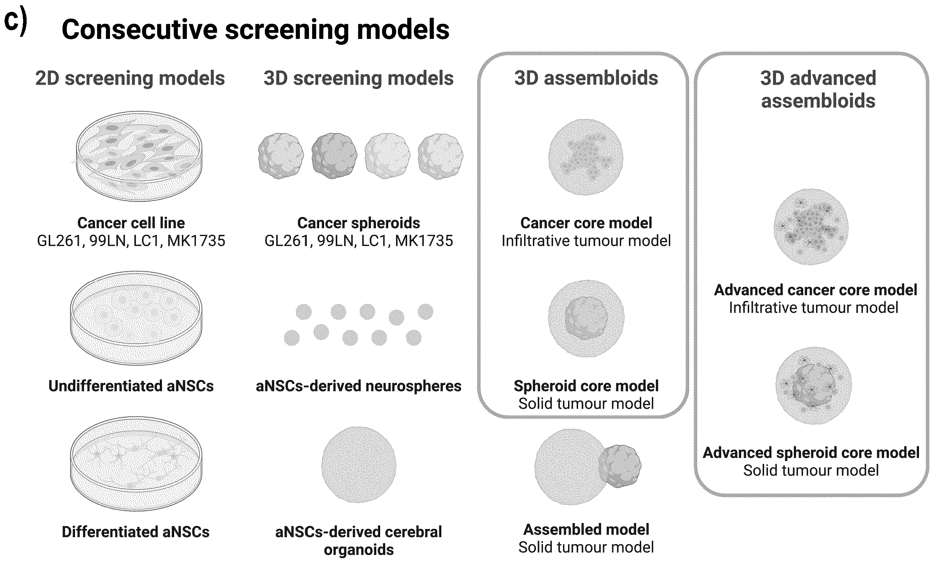

- Fig. 7:: Advancing drug discovery through 3R-compliant aNSC-based models: enhancing *in vitro* to *in vivo* translation. a, Schematic of an optimized iterative hit-to-lead drug discovery process, featuring the integration of 2D neural models for high-throughput neurotoxicity assessments and 3D cerebral organoids and cancer-cell integrated assembloids for medium-throughput combined efficacy and neurotoxicity assessment, minimising the *in vitro* to *in vivo* translation gap. b,c, Overview of consecutive drug screening models with increasing complexity; established drug efficacy screenings are complemented by 2D and 3D neurotoxicity assessments and assembloid screenings. Advanced core models incorporate microglia to boost clinical relevance. Created with BioRender.com.

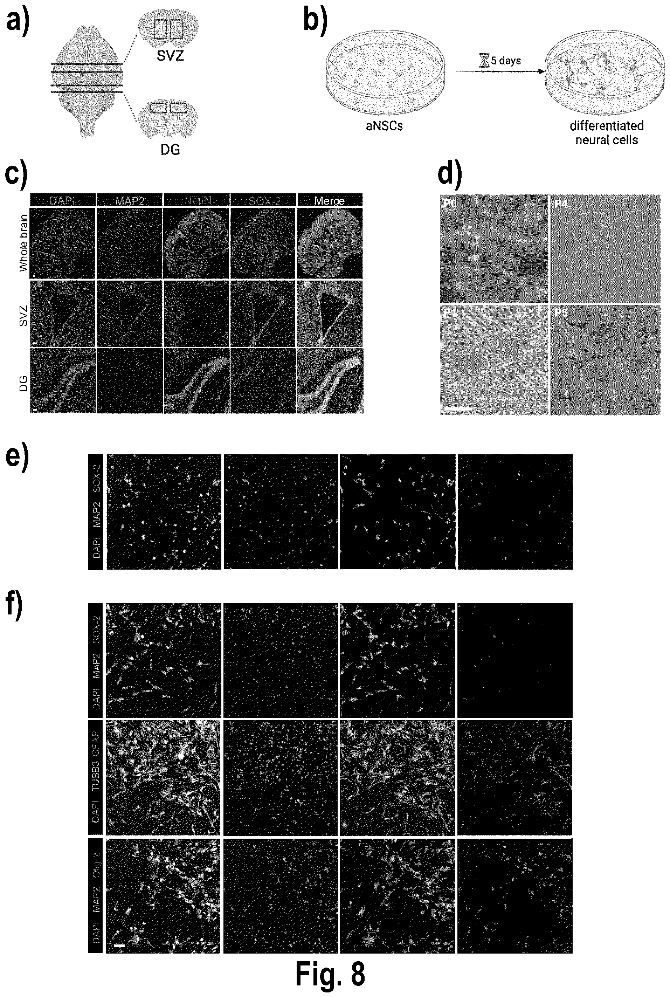

- Fig. 8:: Neurosphere-derived 2D Neural Models. a, Schematic illustration of a murine brain, highlighting the subventricular zone and the dentate gyrus in the hippocampus between the black lines. b, Diagram of aNSCs differentiation into neural cell types in a 2D culture system. c, Immunofluorescence (IF) staining using neural stem cell markers SOX-2 and Nestin and neural differentiation marker NeuN to identify neural stem cells and mature neurons in the lateral ventricle walls of the subventricular zone and the dentate gyrus of the subgranular zone in the hippocampus. Scale bars: 400 µm; 50 µm. d, Brightfield images showing the formation of adult stem cell-derived neurospheres over time in culture. Scale bars: 200; 100 µm; notation 'P' indicates passage number. e and f, IF staining for undifferentiated neural *stem* cells after 24 hours (e) and differentiated aNSCs after 5 days (f) in 2D culture stained for the neural stem cell marker SOX-2, for the neuronal marker MAP2, for the astrocytic marker GFAP and Olig2 as a marker for oli-godendrocyte precursor cells (OPCs) and oligodendrocytes. Scale bar: 50 µm.

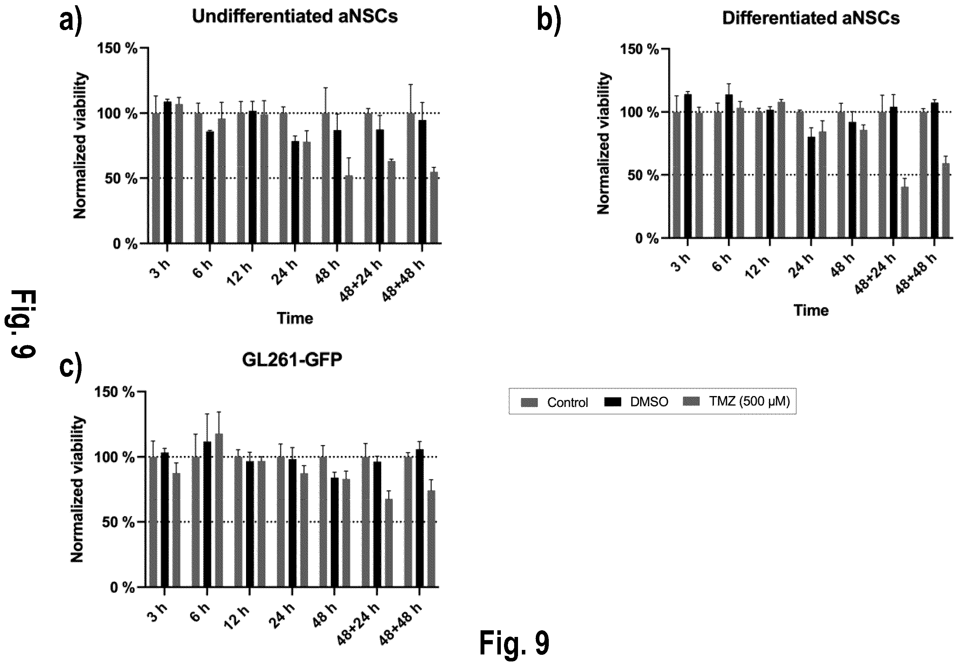

- Fig. 9:: Assessing Drug Neurotoxicity and Efficacy on aNSCs and Glioma Cells. a, Rapid 2D in vitro high-throughput experimental setup for assessing drug effects on aNSCs with high stem cell characteristics. aNSCs were seeded into a monolayer and allowed to attach for 12 hours prior to drug exposure. b, Setup for neurotoxicity assessment in differentiated neurons, astrocytes, and oligodendrocytes. Cells were allowed to differentiate over 3 days before treatment. c, Reference setup using the murine glioma cell line GL261, seeded and allowed to attach for 12 hours before drug exposure. All cells were treated with Temozolomide (TMZ) at a therapeutic dose of 500 µM for 48 hours, followed by a 48-hour recovery period in fresh media. Cell viability was measured at multiple time points using MTS assays, with results referenced against the media-only control. DMSO (1% v/v) served as a negative control.

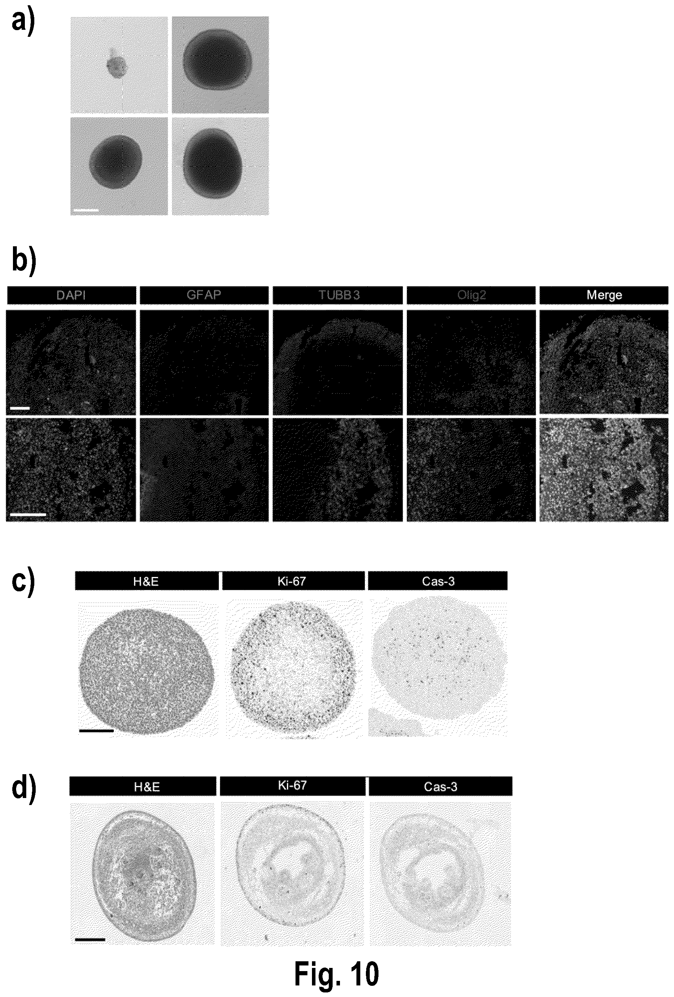

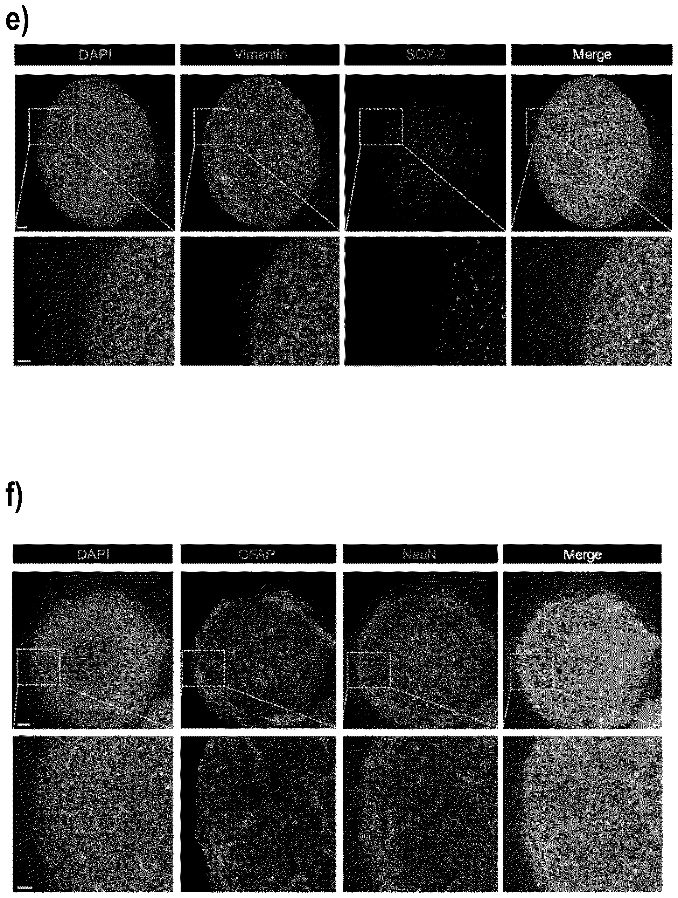

- Fig. 10:: aNSC-derived large Cerebral Organoid Culture. a, Visualization of the growth process for cerebral organoids derived from aNSCs. Scale bar: 200 µm b, Cerebral organoid thin sections showing the expression of neural maturation markers stained for astrocytes (GFAP), neurons (TUBB3) and OPCs as well as oligodendrocytes (Olig2). Scale bar: 200 µm. c and d, Immunohistochemical analysis of thin sections using Ki-67 and an antibody against cleaved caspase-3 to identify highly proliferative regions as well as apoptotic regions at different developmental stages of cerebral organoids: 8-week-old (c) and 12-week-old (d). Scale bar: 300 µm; 500 µm. e and f Immunofluorescence of whole 8-week-old organoids highlighting the expression of neural stem cell markers Vimentin and SOX-2 (e) and differentiation markers such as GFAP and NeuN for astrocytes and neurons (f). Scale bar: 100 µm; 50 µm.

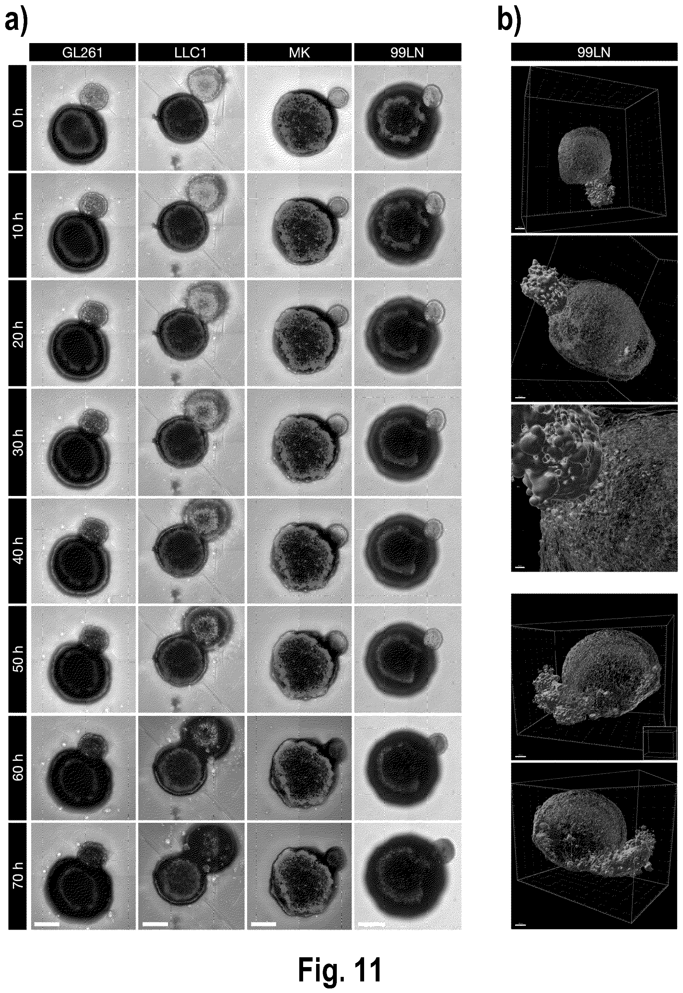

- Fig. 11:: Unveiling the infiltrative Potential of various Cancer Spheroids in Co-culture with aNSC-derived Cerebral Organoids. a, Live-cell imaging of cerebral organoids in co-culture with tumor spheroids derived from primary and secondary brain cancer cell lines: glioma (GL261), lung-to-brain (LLC1), melanoma-to-brain (MK) and breast-to-brain (99LN) over 72 hours. Scale bar: 500 µm. b and c, Three-dimensional representation of a cerebral organoid fused with a breast-to-brain (99LN) spheroid to identify the migration of cancer cells towards the cerebral tissue. Scale bar: 100 µm.

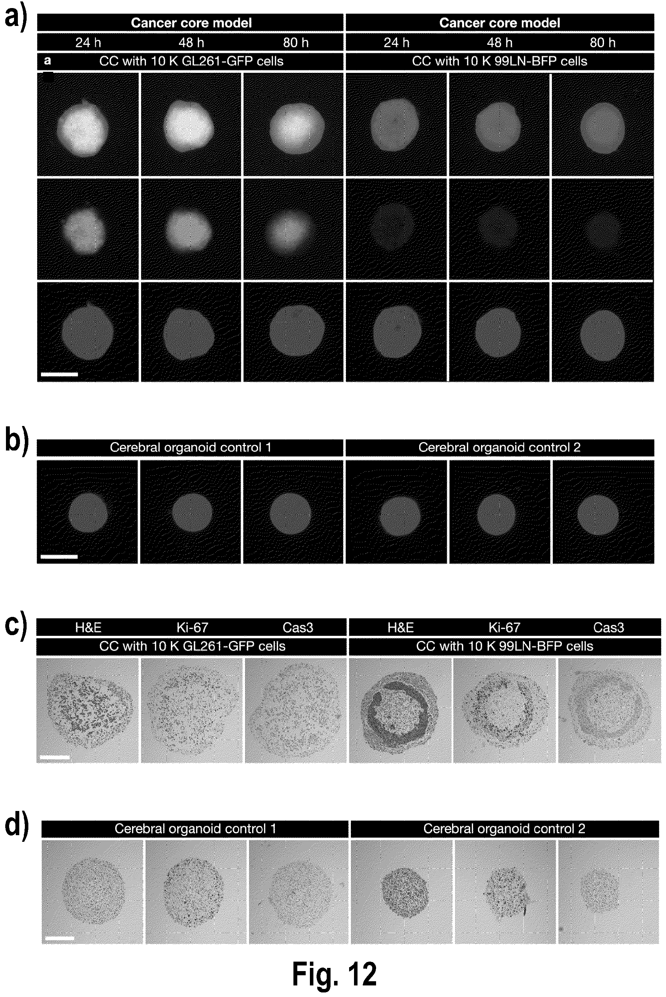

- Fig. 12:: 3D Co-culture Models and Control Cerebral Organoids. a, Real-time visualization of cancer core co-culture interactions using benchtop fluorescence microscopy. b, 3D sphere formation of reseeded cerebral organoids monitored over 72 hours. c, IHC staining shows proliferative cancer cores and infiltrative glioma cells in the GL261-GFP model (left), distinct cancer entities in the breast-to-brain 99LN-BFP model (right), and minimal apoptosis in non-cancerous tissue. d, Control cerebral organoids with distinct 3D spheres, strong proliferation (Ki67), and minimal apoptosis (Caspase-3). Scale bar: 500 µm.

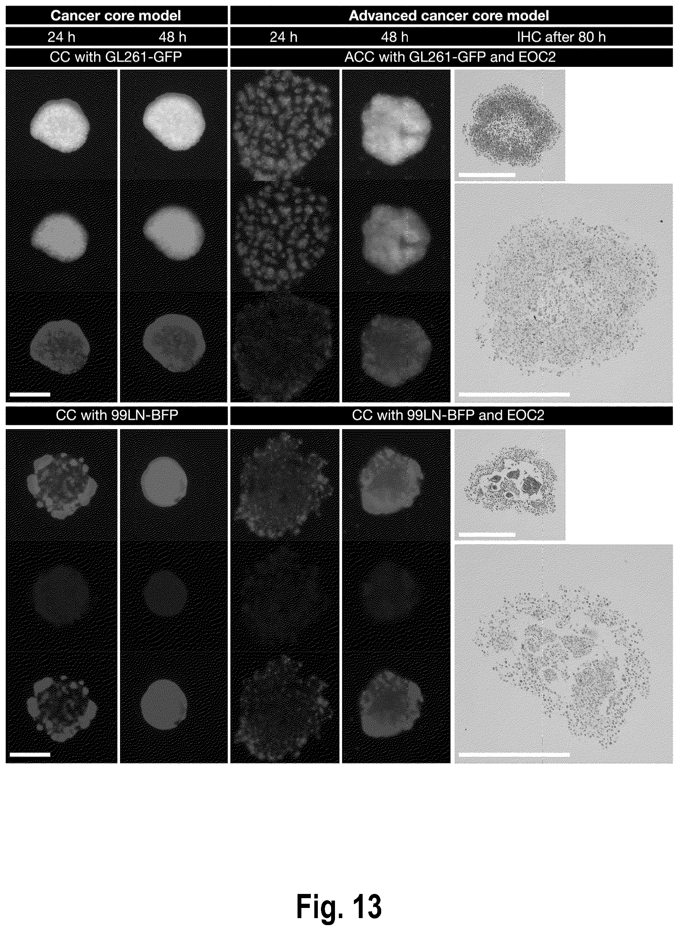

- Fig. 13:: 3D Cancer Core Models with and without Microglia (EOC2). Control models without microglia, showing a 4:1 ratio of cerebral organoid cells to cancer cells (left). Models with microglia (EOC2) integrated at a 4:1:2 ratio of cerebral organoid cells, cancer cells, and microglia (middle). IHC staining shows high EOC2 content within infiltrative tumor mass in the GL261-GFP model (top right) and microglia exclusion in the breast-to-brain 99LN-BFP model (bottom right). Scale bar: 500 µm.

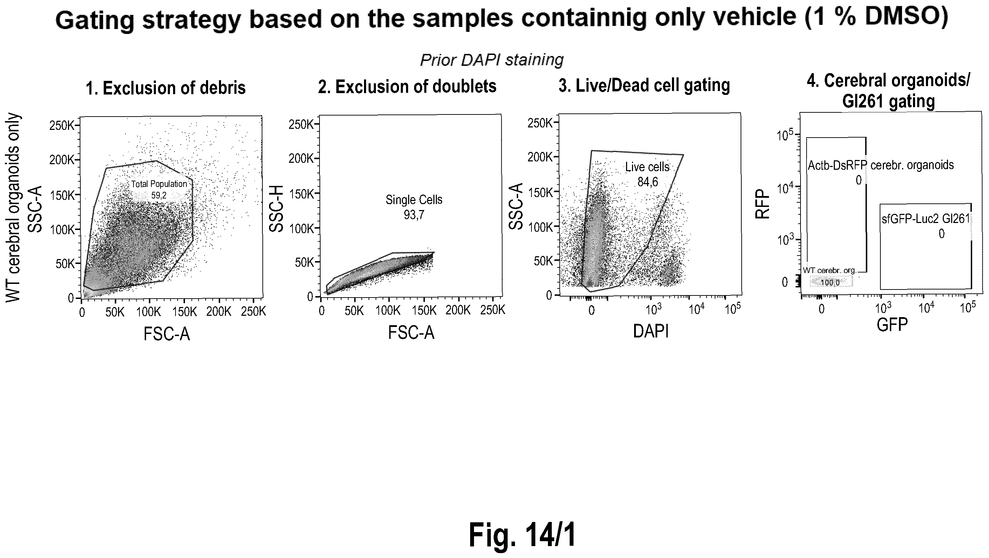

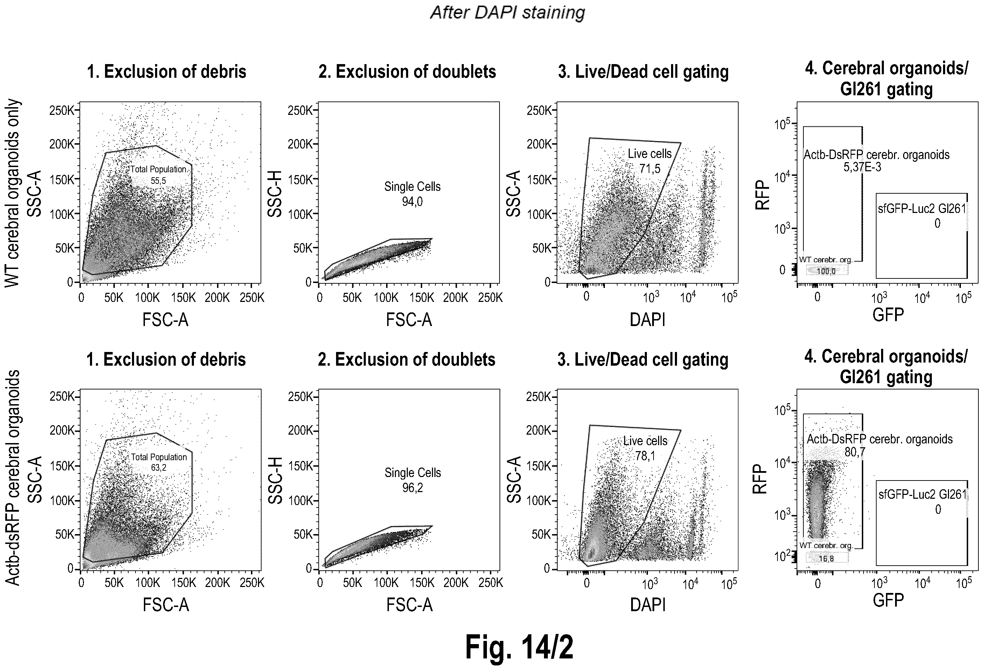

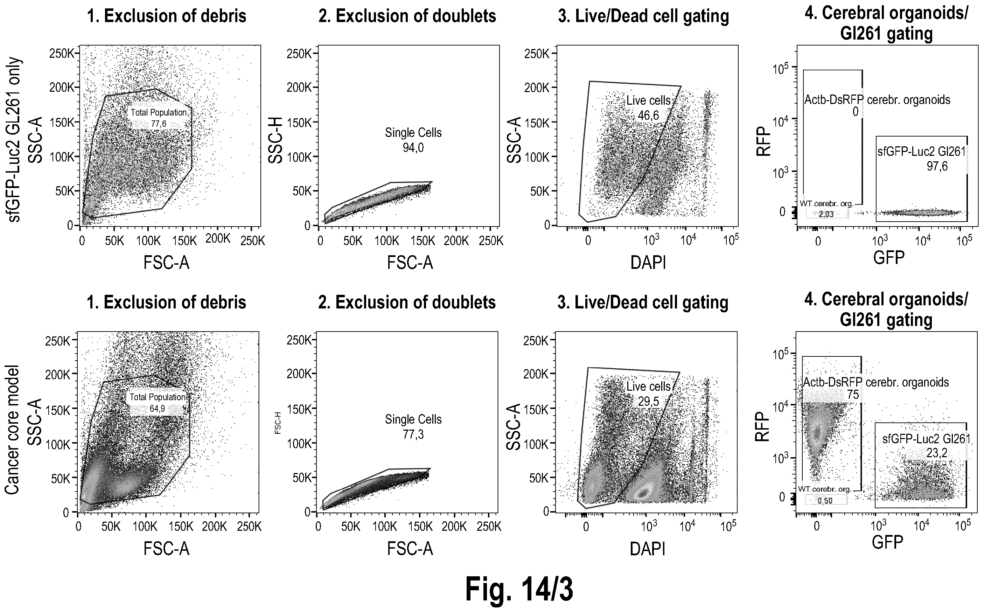

- Fig. 14:: Gating Strategy for FACS-Based Segregation of Viable Cell Populations in 3D Cancer Core Models. Gating strategy using WT cerebral organoids with and without DAPI to identify the live cell fraction (first and second row). RFP-labeled cerebral organoids with DAPI to isolate the live RFP-positive fraction (third row). 2D GL26-GFP cell suspension to identify the live GFP-positive cancer cell fraction fourth row). Application of the gating strategy to the glioma core model to obtain viable subpopulations of non-cancerous organoid cells (RFP-positive, DAPI-negative) and cancerous cells (GFP-positive, DAPI-negative) (last row).

### EXAMPLES

### A. Material and methods

### 1. Harvesting murine adult neural stem cells (aNSCs)

The following procedure for harvesting aNSCs from murine brain tissue carried out:
*Preparation and tissue isolation:* Mice aged 6-24 weeks were eu-thanized and the dissection area, surgical tools and the mouse head were sterilized with 70 % ethanol. The brain was excised and immediately placed in ice-cold dissection buffer (HBSS (Gibco, 14025-126), 2 mM HEPES (Sigma-Aldrich, H3375)). In a laminar flow hood, the hippocampus or subventricular zone (SVZ) was isolated and the dentate gyrus (DG) and SVZ regions were dissected, minced and suspended in dissection buffer on ice.

*Centrifugation and digestion:* The tissue was centrifuged at 200 g, 24°C for 1 minute. The supernatant was discarded and 1 mL of 0.05 % trypsin-EDTA (Gibco, 25300054) was added. The sample was incubated at 37 °C with gentle shaking for 15-20 minutes. Digestion was monitored and extended as necessary, ensuring not to exceed 30 minutes. After digestion, 1 mL of trypsin inhibitor (Sigma-Aldrich, T6522) was added, followed by an additional 20-minute incubation. Trituration was performed carefully using p1000 and p200 pipettes until a single-cell suspension was achieved.

*Washing:* The sample was diluted with 5 mL of *wash* media (Advanced DMEM/F12, Gibco, 12634010; 1 % Pen/Strep, Gibco, 15070063; 1 % GlutaMAX, Gibco, 35050061), then centrifuged at 200 g, 24°C for 5 minutes. Cells were washed twice with 5 mL of wash media, followed by further centrifugation.

*Straining and seeding:* After the final wash, cells were resuspended in 3 mL of aNSC growth media (Neural stem cell basal media (EMD Millipore, SCM003), 1 % B27-A (Gibco, 12587010), 1 % Antibiotic-Antimycotic (Sigma-Aldrich, A5955), 1 % GlutaMAX (Gibco, 35050061), 7.5 µl EGF (100 µg/ml), PeproTech, 315-09); 7,5 µl FGF (100 µg/ml), PeproTech, 100-18B per 25 ml media). The suspension was filtered through a 100 µm cell strainer (Miltenyi Biotec, 130-098-463) and centrifuged again. The resultant cell pellet was resuspended in 1 mL of aNSC growth media and seeded into a single well of a 24-well ULA culture plate (Corning, 3473).

*Incubation and media changes:* Cells were incubated at 37 °C in a humidified 5 % CO₂ incubator. Half of the media was replaced every other day, avoiding disruption of the neurospheres.

This method supports the efficient isolation and initial culture of murine aNSCs for downstream applications.

### 2. Culture of adult neurospheres

This section describes two methods for culturing and passaging neurospheres (from aNSCs), ensuring their maintenance at 50-100 µm in diameter and regular passaging every 2-4 days. The first method utilizes traditional 24-well ULA plates or 25 cm² flasks, while the second offers an alternative using the CERO 3D Incubator & Bioreactor.

### Traditional Culture Method

Collection and enzymatic dissociation: Neurospheres (50-100 µm in diameter) were collected using a p1000 pipette and transferred into a 5-mL tube (Greiner, 622201). Cells were centrifuged at 200 g, 24 °C for 5 minutes. The media was aspirated and 1 mL of 0.05 % trypsin-EDTA (Gibco, 25300054) was added to each well/flask. The suspension was transferred to a 2-mL tube for further processing. The tubes were incubated at 37 °C with slow mixing for 2 minutes. An equal volume of trypsin inhibitor (Sigma-Aldrich, T6522) was added and the cells were triturated first with a p1000 pipette, then with a p200 for finer dissociation.

*Centrifugation and resuspension:* Following trituration, the cell mixture was centrifuged again at 200 g, 24°C for 5 minutes. The supernatant was aspirated the cell pellet was resuspended in aNSC growth media (Neural Stem Cell Basal Media, EMD Millipore, SCM003; 1 % (v/v) B27-A, Gibco, 12587010; 1 % (v/v) Antibiotic-Antimycotic, Sigma-Aldrich, A5955; 1 % (v/v) GlutaMAX, Gibco, 35050061; 7.5 µl EGF (100 µg/ml), PeproTech, 315-09; 7.5 µl FGF (100 µg/ml), PeproTech, 100-18B per 25 ml media).

*Seeding:* Finally, the cells were seeded into a 24-well ULA culture plate (Corning, 3473) at 1 mL per well or into a 25 cm² culture flask (Greiner, 690175) with 3.5-5 mL of aNSCs growth media per flask. Cells were incubated at 37 °C in a 5 % CO2 humidified incubator, with passaging every 2-4 days.

### Alternative Method Using CERO 3D Incubator & Bioreactor

The CERO 3D Incubator & Bioreactor provides a more efficient alternative, allowing larger-scale cultures with the capacity to grow up to 50 million aNSCs per tube in 10 mL of media.

*Procedure:* The cells were passaged following the same initial steps as above to generate a single-cell suspension. After resuspension, the cells were passed through a 70 µm strainer to ensure uniformity and the suspension was transferred into a CERO tube with at least 12 mL of aNSCs growth media (Neural Stem Cell Basal Media, EMD Millipore, SCM003; 1 % (v/v) B27-A, Gibco, 12587010; 1 % (v/v) Antibiotic-Antimycotic, Sigma-Aldrich, A5955; 1 % (v/v) GlutaMAX, Gibco, 35050061; 7.5 µl EGF (100 µg/ml), PeproTech, 315-09; 7.5 µl FGF (100 µg/ml), PeproTech, 100-18B per 25 ml media).

The CERO bioreactor was set to rotate 1 second clockwise and 1 second counterclockwise with continuous motion, providing optimal mixing and preventing cell aggregation during incubation.

### Advantages of CERO Bioreactor.

- *Efficient growth:* The continuous rotation of the CERO system keeps cells in suspension afloat, preventing premature clumping and enabling rapid expansion, with several million cells forming per tube.
- *Neurosphere formation:* After 3-5 days, well-formed neurospheres will develop. Passaging is recommended once neurospheres reach 50-100 µm in diameter, approximately every week.

*Optional cerebral organoid formation:* Larger neurospheres can serve as starting material for cerebral organoid development, streamlining workflows for organoid culture.

### 3. Cultivation of Cerebral Organoids from Adult Neural Stem Cells (aNSCs)

Cerebral organoids were generated from aNSCs using two approaches: an orbital shaker method and the CERO 3D Incubator & Bioreactor for enhanced efficiency and scalability.

### Method 1: Cultivation Using an Orbital Shaker

*Neurosphere Splitting and Cell Quantification:* Neurospheres were dissociated into single-cell suspensions following the procedure outlined in Section A5. Cell numbers were determined using a Neubauer chamber (Hycor, 87144) after staining with Trypan Blue (Carl Roth, CN76.3).

*Organoid Seeding:* aNSCs were seeded at a density of 5,000-20,000 cells per well in a 96-well ULA plate (Nunclon, 174929) in 200 µL of M1 media (Adv. DMEM/F12, Gibco, 12634010; 1 % B27, Gibco, 17504044; 1 % L-Gln, Gibco, 25030-024; 1 % GlutaMAX, Gibco, 35050061; 1 % N-2 supplement, Gibco, 17502048; 1 % Pen/Strep, Gibco, 15070063; 7.5 µl EGF (100 µg/ml), PeproTech, 315-09; 7.5 µl FGF (100 µg/ml), PeproTech, 100-18B per 25 ml media).

*Medium Replacement:* After 72 hours, 100 µL of M1 media was aspirated and replaced with M2 media (Adv. DMEM/F12, 1 % B27, 1 % L-Gln or GlutaMAX, 1 % Pen/Strep, 1 % N-2 supplement, 1 % Pen/Strep, 5 µl EGF (100 µg/ml), PeproTech, 315-09; 5 µl FGF (100 µg/ml), PeproTech, 100-18B per 25 ml media). After another 72 hours, 100 µL of M2 media was replaced with M3 media (Adv. DMEM/F12, 1 % B27, 1 % L-Gln or GlutaMAX, 1 % Pen/Strep, 2.5 µl EGF (100 µg/ml), PeproTech, 315-09; 2.5 µl FGF (100 µg/ml), PeproTech, 100-18B per 25 ml media).

*Organoid Transfer and Maintenance:* After 72 hours in M3 media, organoids were transferred to 6-well suspension plates (Greiner, 657185) with up to 3 mL of M3 media per well. Media changes were conducted every 72 hours for continued growth and maintenance.

### Method 2: Cultivation Using the CERO 3D Incubator & Bioreactor

*Overview:* The CERO 3D Incubator & Bioreactor offers an alternative to traditional methods, promoting round, uniform organoid growth with reduced hands-on time and increased scalability. The larger culture volume also minimizes the frequency of media changes.

*Pre-seeding in ULA Plates:* A single-cell suspension from neurospheres was prepared as per the procedure in A.5. Cell counts were determined using a Neubauer chamber after Trypan Blue staining. 5,000-20,000 cells were seeded per well in a 96-well ULA plate (Nunclon, 174929) in 200 µL of M1 media (same composition as above).

*Transfer to CERO Tubes:* After 3 days, organoids from the ULA plates were transferred to CERO tubes with at least 12 mL of M2 media (Adv. DMEM/F12, 1 % B27, 1 % L-Gln, 1 % GlutaMAX, 1 % N-2 supplement, 1 % Pen/Strep, 5 µl EGF (100 µg/ml), PeproTech, 315-09; 5 µl FGF (100 µg/ml), PeproTech, 100-18B per 25 ml media).

*Media Changes and Incubation:* Media changes were performed twice per week, replacing half of the media or adding 3 mL of fresh media if the medium retains a healthy color. After 1 week, the media was switched to M3 media (same composition as above) for all subsequent media changes. Organoids grew up to 2 mm in diameter within 3 months, with long-term cultivation of over 5 months being possible.

### Incubation Parameters:

- For initial growth, set the CERO bioreactor to rotate 1 second clockwise and 1 second counterclockwise continuously to ensure proper mixing.
- Once organoids reach 250 µm in diameter, adjust the rotation to 2 seconds clockwise, pause for 1 second, then 2 seconds counterclockwise, with a 1-second pause to maintain optimal culture conditions as organoids grow larger.

### Alternative Method: Direct Neurosphere Outgrowth into Organoid Culture

An alternative approach for simplifying organoid generation involves allowing aNSCs to transition directly from neurospheres to organoids.

*Procedure:* Once neurospheres reach 100 µm in diameter, replace the neurosphere media with M1 media. After 1 week, switch to M2 media during the next media change. After another week, switch to M3 media for all subsequent changes.

This method reduces hands-on time and materials while improving reproducibility.

### 4. Cultivation and Maintenance of Murine Cell Lines

Murine cell lines were cultured under controlled conditions to assess cellular responses, following established cell culture procedure.

*Culture Maintenance:* Cells were incubated at 37 °C in a humidified atmosphere with 5 % CO₂. At 80 % cell confluency, cells were passaged to maintain optimal growth conditions, e.g., nutrient levels and cell health.

*Thawing Procedure:* Frozen cell lines were rapidly thawed at 37°C, then transferred to a 15 mL Falcon tube (Greiner, 188271) containing 4 mL of pre-warmed adv. DMEM compl. media (Adv. DMEM/F12, Gibco, 12634010; 10 % FBS, PAN-Biotech, P30-3302; 1 % L-Gln, Gibco, 25030-024; 1 %Pen/Strep, Gibco, 15070063). After centrifugation at 1500 rpm for 3 minutes at room temperature, the supernatant was aspirated and the cell pellet was resuspended in fresh adv. DMEM compl. media before being seeded into a T25 flask (Greiner, 690175).

*Passaging Procedure:* Confluent cells were passaged by removing the media and incubating the cell layer with 0.05% trypsin-EDTA (Gibco, 25300054) for 2-5 minutes at 37°C. The reaction was neutralized with DMEM compl. media (DMEM, Gibco, 21969-035; 10% FBS, PAN-Biotech, P30-3302; 1 % L-Gln, Gibco, 25030-024; 1 %Pen/Strep, Gibco, 15070063) and the cells were centrifuged at 1500 rpm for 3 minutes at 4°C. The cell pellet was resuspended in fresh media, with part of the suspension transferred to a new flask for continued culture and the rest reserved for experimental assays.

*Cryopreservation:* For long-term storage, cells were split as per the passaging procedure. The final cell pellet was resuspended in adv. DMEM compl. media supplemented with 10 % DMSO (Sigma-Aldrich, 67-68-5), aliquoted into cryovials (Greiner, 126263) and gradually cooled to -80°C before transfer to liquid nitrogen for storage. This procedure ensured the reliable cultivation, expansion and preservation of murine cell lines for use in experimental assays.

### 5. Cancer core and spheroid core models

This method describes the establishment of Cancer Core and Spheroid Core models, integrating cancer cells or pre-formed spheroids with differentiated cerebral organoids to represent distinct tumor microenvironments (TMEs). These models are highly reproducible, allowing for precise control of both healthy cerebral and cancer cell populations, ensuring consistent cell ratios across replicates.

### Cancer Core Model

The *Cancer Core model* integrates single cancer cells into cerebral organoids to ensure controlled tumor growth within the organoid. This model ensures consistent cancer and cerebral cell populations across replicates and allows the generation of specific cancer core patterns depending on the seeded cell ratios and cell backgrounds.

### Tested Cell Ratios:

The following cell ratios were tested to study how varying cancer cell loads influence core formation:

**Table 1: Tested cell ratios to study how varying cancer cell loads influence core formation**

| **Cerebral Organoids** | **Cancer Cells** | Cancer Core Ratio | Cancer Core Formation |
|---|---|---|---|
| | Cell number per well | | |
| Cell number per well | | | |
| **20,000** | 10,000 | 2:1 | Mosaic-like pattern with cancer cells and non-cancerous cells throughout the whole model |
| **40,000** | 10,000 | 4:1 | Cancer core unit with high cancer cell density |
| **40,000** | 20,000 | 2:1 | Mosaic-like pattern with cancer cells and non-cancerous cells throughout the whole model |
| **80,000** | 20,000 | 4:1 | Cancer core unit with high cancer cell density |
| **100,000** | 1,000 | 100:1 | Patch-like pattern with cancer cells and non-cancerous cells in the core area and well-defined cerebral 'shell' |
| **100,000** | 5,000 | 20:1 | Mosaic-like pattern with cancer cells and non-cancerous cells in the core area and well-defined cerebral 'shell' |
| **100,000** | 10,000 | 10:1 | Cancer core unit with high cancer cell density with larger cerebral 'shell' |
| **100,000** | 25,000 | 4:1 | Cancer core unit with high cancer cell density |

These tested cell ratios led to distinct *Cancer Core model* patterns, influenced by the cell ratio (healthy cerebral cells vs. pathological cancer cells):
- *2:1 ratio* -> Produces a mosaic-like pattern with cancer cells distributed throughout the structure and no well-defined cerebral 'shell'.
- *4:1 ratio* -> Forms a core unit pattern with a clearly defined cerebral 'shell'.
- *10:1 ratio* -> Generates a dense cancer core unit with a high concentration of cancer cells and a larger cerebral 'shell'.
- *20:1 ratio* -> Creates a mosaic-like pattern where both cancerous and non-cancerous cells were mixed within the core area, surrounded by a well-defined cerebral 'shell'.
- *100:1 ratio* -> Produces a patch-like pattern with cancer cells foci distributed throughout the structure, surrounded by a well-defined cerebral 'shell'.

### Influence of Cancer Cell Type:

The cancer core formation also strongly depends on the biological background of the cancer cell line:
- *Glioma model (GL261):* Exhibits a highly infiltrative pattern, with cancer cells spreading widely throughout the cerebral organoid, mimicking the invasive nature of gliomas observed clinically.
- *Breast-to-brain metastasis model (99LN-BrM 80478*/*79, short 99NL):* Tends to form well-defined, localized cancer regions within the cerebral organoid, reflecting the distinct tumor morphology seen in breast-to-brain metastases.
- *Lung-to-brain metastasis model (LLC1):* Highly invasive, with fast-growing cancer areas that resemble metastatic cancer spread in the brain.
- *Melanoma-to-brain metastasis model (MK1735):* Tends to form more localized and well-defined cancer areas within the organoid structure.

These findings highlight the versatility of the Cancer Core Model in replicating a variety of tumor microenvironments, allowing for precise simulation of cancer behavior based on both cell ratio and cancer type.

*Cancer Core Formation Procedure:* Cancer cells (e.g., GL261-GFP, 99LN-BFP, LLC1-GFP, or MK-BFP) were prepared from 2D culture following standard passaging procedures. Cells were counted using a Neubauer chamber and viability was assessed with Trypan Blue. The cancer cells were resuspended in M3 media (Adv. DMEM/F12, Gibco, 12634010; 1 %B27, Gibco, 17504044; 1 % L-Gln, Gibco, 25030-024; 1 %GlutaMAX, Gibco, 35050061; 1 %Pen/Strep, Gibco, 15070063; 2.5 µl EGF (100 µg/ml), PeproTech, 315-09; 2.5 µl FGF (100 µg/ml), PeproTech, 100-18B) per 25 ml media).

*Organoid Dissociation for Cancer Core Formation:* In parallel, pre-differentiated cerebral organoids (grown for a minimum of 1 month) were enzymatically dissociated to create a single-cell suspension to integrate with cancer cells. In detail, fifty to one hundred cerebral organoids (diameter >300 µm) were transferred into a 15 mL Falcon tube and centrifuged at 200 g for 2 minutes. The media was aspirated and 1-2 mL of 0.05% trypsin-EDTA (Gibco, 25300054) was added. The organoid suspension was incubated at 37 °C on an orbital shaker (Thermo Fisher, 88881101) for 2 minutes. After incubation, the organoids were gently triturated with a p1000 pipette, followed by a p200 pipette, until a uniform single-cell suspension was achieved. To stop the enzymatic reaction, 1 mL of trypsin inhibitor (Sigma-Aldrich, 6522) was added. The suspension was centrifuged again at 200 g for 2 minutes and the pellet was resuspended in 1-2 mL of M3 media. The resulting suspension was filtered through a 100 µm EASYstrainer (Miltenyi Biotec, 130-098-463) to ensure uniformity. Cells were counted using a Neubauer chamber and viability was assessed with Trypan Blue.

*Final Cancer Core Assembly:* Depending on the desired cerebral organoid-to-cancer cell ratio, mixtures were prepared using ratios ranging from 2:1 to 20:1 (cerebral organoid cells to cancer cells). These mixtures were distributed into a 96-well ULA plate at 200 µL per well. The plates were incubated at 37 °C in a humidified 5% CO₂ incubator on an orbital shaker for 72 hours, allowing the cancer cells to integrate with the organoid-derived cells and form distinct Cancer Core Models. The resulting patterns-ranging from mosaic-like distributions to well-defined cancer core units-depended on the specific ratio used and the cancer cell type.

### Spheroid Core Model

The *Spheroid Core Model* embeds pre-formed spheroids into cerebral organoids to represent distinct hypoxia-driven TMEs for medium-throughput drug efficacy and neurotoxicity testing.

*Spheroid Culture:* Spheroids were generated from a single-cell suspension using the cell passaging method as described in Section 3. Cells were counted using a Neubauer chamber (Hycor, 87144) and confirming viability with Trypan Blue (Carl Roth, CN76.3). Cells were suspended in spheroid-specific media (DMEM compl. or DMEM, Gibco, 21969-035; 10% FBS, PAN-Biotech, P30-3302; 1 % L-Gln, Gibco, 25030-024; 1 %Pen/Strep, Gibco, 15070063 and optionally 12.5 ng/mL EGF, PeproTech, 315-09). Cells were seeded into U-shaped, 96-well ULA plates (Nunclon, 174929) at a density of 10,000 to 40,000 cells per well in 200 µL of media and incubated at 37 °C in a humidified 5 % CO₂ incubator for 48-72 hours to promote spheroid formation.

*Spheroid Core Formation:* After 48-72 hours of spheroid culture, pre-formed spheroids were transferred into a fresh 96-well ULA plate.

*Organoid Dissociation for Spheroid Core Formation:* As with the Cancer Core Model, pre-differentiated cerebral organoids were enzymatically dissociated to create a single-cell suspension for integration with the spheroids. 50-100 organoids (diameter >300 µm) were transferred into a 15 mL Falcon tube and centrifuged at 200 g for 2 minutes. The media was aspirated and 1-2 mL of 0.05% trypsin-EDTA (Gibco, 25300054) was added. The organoid suspension was incubated at 37 °C on an orbital shaker for 2 minutes. The organoids were then triturated with a p1000 and p200 pipette, until a uniform single-cell suspension was achieved. Trypsin inhibitor (Sigma-Aldrich, T6522) was added, followed by centrifugation and resuspension in M3 media. The suspension was filtered through a 100 µm EASYstrainer to ensure uniformity.

*Final Spheroid Core Assembly:* To generate spheroid cores, 100,000 cerebral cells were combined with a single cancer spheroid per well in 200 µL of M3 media in a U-shaped 96-well ULA plate. The plates were incubated at 37 °C in a humidified 5 % CO₂ incubator. After 24 hours, plates were transferred onto an orbital shaker for additional 48 hours to promote formation of the spheroid core model.

### Monitoring and Clinical Relevance

Both the Cancer Core and Spheroid Core models were monitored using a bench to microscope, e.g., EVOS^{™} Digital Color Fluorescence microscopy (Invitrogen) and BioTek Cytation C10 Confocal Imaging Reader (Agilent), to track the interaction and integration of cancer cells or spheroids with organoid-derived cells.

These models exhibit reproducible morphologies, overcoming the variability of traditional models where cancer cell uptake and tumor formation are inconsistent. By precisely controlling the number of healthy cerebral cells - including neural stem cells, progenitor cells, neurons, astrocytes and oligodendrocytes - this protocol ensures reproducibility across all models. The controlled cancer cell uptake further enhances the reproducibility, allowing for consistent cancer cell populations across replicates.

### 6. Advanced Cancer Core and Advanced Spheroid Core models

This method outlines the development of Advanced Cancer Core and Advanced Spheroid Core models, incorporating immune cells, particularly microglia, alongside cancer cells or pre-formed spheroids. These models more accurately represent the tumor microenvironment (TME), enabling a detailed study of tumor-immune interactions and cancer progression.

### Advanced Cancer Core Model

The Advanced Cancer Core model integrates tumor cells, dissociated cerebral organoid cells, and immune cells - particularly microglia - within a cerebral organoid structure. This model builds on the Cancer Core model by incorporating immune cells, providing a more realistic simulation of tumor-immune dynamics. It ensures reproducibility and maintains consistent cell densities, while better reflecting the complexity of the TME.

*Tested Cell Ratios:* The following cell ratios were tested, all leading to defined model formation. Unlike the Cancer Core, the Advanced Core initially shows a mosaic-like pattern at 24 hours, with the cerebral shell closing over the next 48 hours to form the Advanced Cancer Core.

**Table 2: Tested cell ratios**

| **Cerebral Organoids (cell number per well)** | **Cancer Cells (cell number per well)** | **Microglia Cells (cell number per well)** | **Ad. Cancer Core Ratio** |
|---|---|---|---|
| **20,000** | 10,000 | 10,000 | 2:1:1 |
| **40,000** | 10,000 | 20,000 | 4:1:2 |
| **40,000** | 20,000 | 20,000 | 2:1:1 |
| **80,000** | 20,000 | 40,000 | 4:1:2 |

*Influence of Cancer Cell Type:* The advanced cancer core formation depends highly on the biological background of the cancer cell line:
- *Glioma Model (GL261):* Exhibits a highly infiltrative pattern with substantial microglia presence within the tumor. This mimics the clinical observation where approximately 30% of glioblastoma tumor mass consists of microglia.
- *Breast-to-Brain Metastasis Model (99LN):* The advanced core exhibits less defined tumor borders compared to the standard 99LN Cancer Core. As seen in clinical samples, microglia are found predominantly at the tumor periphery, leading to peripheral micro-gliosis and microglia exclusion within the tumor tissue.

### Advanced Cancer Core Formation Procedure

Cancer cells (e.g., GL261-GFP, 99LN-BFP) were cultured using standard passaging techniques. Microglia (EOC2 cells) were cultured separately in DMEM compl. media supplemented with Interleukin-34 (IL-34) and TGF-β to maintain cell viability.

*Simultaneous Preparation of Single-Cell Suspensions:* Cerebral organoids (diameter >300 µm, grown for at least 1 month) were transferred into a 15 mL Falcon tube and centrifuged at 200 g for 2 minutes. The media was aspirated, and the organoids were resuspended in 1-2 mL of 0.05% trypsin-EDTA (Gibco, 25300054). They were incubated at 37 °C on an orbital shaker for 2 minutes. Organoids were then gently triturated using a p1000 pipette, followed by a p200 pipette, until a uniform single-cell suspension was achieved. The reaction was stopped by adding trypsin inhibitor (Sigma-Aldrich, T6522), and the suspension was centrifuged at 200 g for 2 minutes. The pellet was resuspended in modified M3 media (containing adv. DMEM/F12, 1% B27, 1% L-Glutamine, 1% GlutaMAX, 1% Pen/Strep, 2.5 µl EGF (100 µg/mL), 2.5 µl bFGF (100 µg/mL), 50 µl IL-34 (100 µg/mL) and 2.5 µl TGF-β (50 µg/mL) per 25 ml M3 media). Cancer cells were detached using 0.05% trypsin-EDTA (Gibco, 25300054), while microglia (EOC2 cells) were detached using a cell scraper. Both were resuspended in modified M3 media (containing IL-34 and TGF-β). All cell types were counted using Neubauer chambers, and their viability was assessed using Trypan Blue exclusion.

*Mastermix Preparation:* A mastermix was prepared according to the desired cell ratios (e.g., 2:1:1, 4:1:2) of cerebral organoid cells, cancer cells, and microglia. The mastermix was given into a U-bottom 96-well ultra-low attachment (ULA) plate at 200 µL per well. The M3 media used was supplemented with IL-34 and TGF-β to support microglia viability and function.

*Seeding and Incubation:* After seeding, the plates were immediately placed on an orbital shaker inside a 37 °C incubator with 5% CO₂. The plates were shaken at 100 rpm for 72 hours to promote Advanced Cancer Core formation.

### Advanced Spheroid Core Model

The Advanced Spheroid Core model incorporates pre-formed cancer spheroids, dissociated cerebral organoid cells, and immune cells (e.g., microglia) to create a tumor microenvironment that mimics brain tumor-immune interactions. This model allows for the investigation of the dynamic interactions between cancer cells, neurons, and immune components, providing a platform for preclinical testing and mechanistic studies of tumor-immune behavior.

*Cancer Spheroid Generation:* Cancer spheroids were generated from a single-cell suspension of cancer cells following the method outlined in Section 4. Briefly, cancer cells were seeded into U-bottom ultra-low attachment (ULA) 96-well plates at a density of 10,000 to 40,000 cells per well, with 200 µL of spheroid-specific media (DMEM compl. or DMEM, 10 % FBS, 1 % L-Glutamine, 1 % Penicillin-Streptomycin). The plates were incubated at 37°C in a 5% CO₂ incubator for 48-72 hours, allowing the cancer cells to aggregate and form stable spheroids.

*Dissociation of Cerebral Organoids:* In parallel, cerebral organoids (grown for at least 1 month, diameter >300 µm) were enzymatically dissociated into a single-cell suspension. This involved centrifugation of the organoids at 200 g for 2 minutes, followed by resuspension in 0.05% trypsin-EDTA. Organoids were incubated at 37 °C for 2 minutes and gently triturated with p1000 and p200 pipettes until a uniform suspension was achieved. The enzymatic reaction was stopped with trypsin inhibitor, and the suspension was filtered through a 100 µm strainer to ensure uniformity. The final suspension was resuspended in modified M3 media containing Advanced DMEM/F12, 1% B27, 1% L-Glutamine, 1% GlutaMAX, 1% Pen/Strep, 2.5 µl EGF (100 µg/ml),, 2.5 µl bFGF (100 µg/ml), 50 µl IL-34 (100 µg/mL), and 2.5 µl TGF-β (50 µg/mL) per 25 ml media to support immune cell function and neuronal health.

*Preparation of Microglia:* Microglia (EOC2 cells) were cultured separately in DMEM complete media supplemented with IL-34 and TGF-β to enhance their viability and functionality. The cells were detached using a cell scraper and counted using a Neubauer chamber. Viability was assessed with Trypan Blue exclusion, and the microglia were resuspended in modified M3 media to match the concentration required for spheroid core formation.

*Spheroid Core Assembly.* For the Advanced Spheroid Core model, the following components were combined in each well:
- 100,000 dissociated cerebral cells (derived from organoids),
- 1 pre-formed cancer spheroid (ranging from 10,000 to 40,000 cells per spheroid), and
- A defined number of microglia (typically 10,000, 20,000, or 40,000 cells per well, depending on the experimental design).

These components were added to each well of a ULA 96-well plate, with 200 µL of supplemented M3 media to maintain cell viability and promote integration. Plates were incubated at 37 °C in a humidified 5 % CO₂ atmosphere for 24 hours without shaking to allow initial cell interaction and spheroid stabilization.

*Incubation and Shaking:* After 24 hours, the plates were transferred to an orbital shaker (inside the 37 °C incubator) to promote further mixing, integration, and co-culture of the spheroid with the dissociated organoid cells and microglia. The shaking was continued for an additional 48 hours to facilitate the formation of a coherent spheroid core model, replicating the tumor microenvironment.

### Monitoring and Clinical Relevance

Both the Advanced Cancer Core and Advanced Spheroid Core models were monitored using fluorescence microscopy (e.g., EVOS^{™} Digital Color Fluorescence, Invitrogen or BioTek Cytation C10 Confocal Imaging Reader, Agilent) to observe the integration of immune cells, tumor cells, and neuronal cells.

*Microglia's Role in Tumor Progression:* Microglia play a pivotal role in brain tumor progression, invasion, and immune response. In this model, microglia's interaction with cancer cells can be observed. Amongst others, this allows researchers to explore whether microglia can be reprogrammed into antitumor phenotypes, offering insights into novel therapeutic strategies.

*Applications in Drug Screening and Immunotherapy:* These advanced models provide a more physiologically relevant platform for preclinical drug testing. They allow for the evaluation of both anticancer agents and immunotherapies, revealing how treatments affect not only tumor cells but also interplay of treatment regimens and the response/impact of the immune compartment. This dual focus improves the predictive accuracy of therapeutic efficacy in human brain cancers. The ability to monitor immune-cancer interactions also highlights potential immune-modulating effects of drugs, which can be critical in developing combination therapies. By mimicking brain tumor microenvironments, these models help bridge the gap between traditional 2D cultures and *in vivo* systems, enhancing the reliability of preclinical studies.

### 7. Neurosphere culture

The following protocol explains the steps of the aNSC passaging workflow, as shown in Figure 1.

### Passaging of Neurospheres (from aNSCs)

1. Use a p1000 pipette to collect all aNSCs (50-100 µm in diameter) and transfer them into a 5 mL tube. Centrifuge at 200 g, 24 °C for 5 minutes and carefully aspirate the media.
2. Add 1 mL of 0.05 % trypsin-EDTA (Gibco, 25300054) to each tube and incubate at 37 °C with gentle mixing for 2 minutes.
3. Add an equal volume of 1 mg/mL trypsin inhibitor (Sigma-Aldrich, T6522), then triturate the cells to a single-cell suspension using a p1000 pipette. For optimized dissociation, triturate with a p200 pipette.
4. Centrifuge the mixture at 200 g, 24 °C for 5 minutes. Carefully aspirate the supernatant.
5. Resuspend the cell pellet in 1-3.5 mL of aNSC growth media (Neural Stem Cell Basal Media (EMD Millipore, SCM003), 1 % B27-A, 1 % Antibiotic-Antimycotic, 1 % GlutaMAX, 7.5 µl EGF (100 µg/ml), PeproTech, 315-09 and 2.5 µl FGF (100 µg/ml), PeproTech, 100-18B per 25 ml media).
6. Seed aNSCs into a 24-well ULA culture plate (Corning, 3473) with 1 mL per well or into a 25 cm² cell culture flask with 3.5-5 mL of media per flask.
7. Incubate at 37 °C in a humidified 5 % CO₂ incubator until the next passaging.

### Alternative Method Using CERO 3D Incubator & Bioreactor

The CERO 3D Incubator & Bioreactor offers a more efficient alternative to the traditional 24-well ULA plates or 25 cm² flasks. It allows for significantly larger cell cultures, with the capacity to handle more than 20 x the number of aNSCs per unit.

### Procedure:

1. After aNSCs isolation and culture establishment, follow the same passaging steps described above (Steps 1-5) to generate a single-cell suspension.
2. Once the final cell pellet is resuspended, pass the cell suspension through a 70 µm cell strainer to ensure a uniform single-cell mixture.
3. Transfer the single-cell suspension into a fresh CERO tube with a minimum of 12 mL of neurosphere media.

*Incubation Parameters:* Set the CERO bioreactor to rotate 1 second clockwise and 1 second counterclockwise, with no breaks in between to maintain optimal mixing and prevent cell aggregation.

### Advantages of CERO Bioreactor.

- Efficient Growth: Unlike plates or flasks, the continuous rotation in the CERO system keeps cells in suspension, preventing early clumping. This enables potent cell growth, allowing several million cells to grow per tube (up to 50 million cells in 10 mL of media).
- Neurosphere Formation: After a few days (up to 5), aNSCs will begin to form well-shaped neurospheres. When maintaining stem cell cultures, passage the neurospheres once they reach a diameter of 50-100 µm (approximately every week).
- Optional Cerebral Organoid Formation: If larger neurospheres are allowed to grow in this system, they can be used as the starting point for cerebral organoid formation, further simplifying culture workflows.

### Troubleshooting

*1. Air Bubble-Associated Decline in aNSC Viability:*
   ∘ Cause: Air bubbles introduced during pipetting can reduce cell viability.
   ∘ Solution (Standard Method): Pipette gently to minimize air bubble formation.
   ∘ Solution (CERO): The closed environment of the CERO system reduces air bubble formation, especially during media changes.
2. *Trypsinization-Associated Decline in aNSC Viability:*
   ∘ Cause: Prolonged exposure to trypsin-EDTA can damage cell structures.
   ∘ Solution (Both Methods): Limit trypsin-EDTA incubation to no more than 2 minutes, closely monitoring cell dissociation.
3. *High Cell Density-Associated Decline in Viability:*
   ∘ Cause: Overcrowding reduces nutrient availability and impairs cell growth.
   ∘ Solution (Standard Method): Distribute cells evenly across wells or flasks and ensure sufficient media. Expand to additional wells or flasks as needed.
   ∘ Solution (CERO): The large media volume and continuous rotation in the CERO system prevent overcrowding and maintain nutrient distribution.
4. *Neurosphere Size-Associated Decline in Viability:*
   o Cause: Neurospheres larger than 100 µm in diameter experience poor nutrient and oxygen diffusion to the core.
   ∘ Solution (Standard Method): Passage neurospheres once they reach 50-100 µm to prevent core depletion.
   ∘ Solution (CERO): The optimized suspension environment in the CERO system supports even nutrient distribution, but regular passaging is still recommended when neurospheres reach 50-100 µm in size.

### 8. Cerebral organoid culture

The following protocol details the steps required for cultivating cerebral organoids, as shown in Figure 2.

### Culturing of aNSCs-derived Cerebral Organoids (utilizing an Orbital Shaker)

1. Prepare a single cell suspension from neurospheres following the neurosphere culture protocol (3.2).
2. Determine cell number by counting cells using a Neubauer chamber after brief Trypan Blue (Carl Roth, CN76.3) staining.
3. Seed 5,000-20,000 cells per well in a 96-well ULA plate (Nunclon, 174929) in 200 µL of M1 media (Adv. DMEM/F12 (Gibco, 12634010), 1 % (v/v) B27 (Gibco, 17504044), 1 % (v/v) L-Gln (Gibco, 25030-024), 1 % (v/v) GlutaMAX (Gibco, 35050061), 1 % (v/v) N-2 supplement (Gibco, 17502048), 1 % (v/v) Pen/Strep (Gibco, 15070063), 7.5 µl EGF (100 µg/ml), PeproTech, 315-09; 7.5 µl FGF (100 µg/ml), PeproTech, 100-18B) per 25 ml media).
4. After 72 hours, aspirate 100 µL of media and replace with M2 media (Adv. DMEM/F12 (Gibco, 12634010), 1 % (v/v) B27 (Gibco, 17504044), 1 % (v/v) L-Gln (Gibco, 25030-024), 1 % (v/v) GlutaMAX (Gibco, 35050061), 1 % (v/v) N-2 supplement (Gibco, 17502048), 1 % (v/v) Pen/Strep (Gibco, 15070063), 5 µl EGF (100 µg/ml), PeproTech, 315-09; 5 µl FGF (100 µg/ml), PeproTech, 100-18B per 25 ml M2 media).
5. After another 72 hours, aspirate 100 µL of the M2 media and add 100 µL of M3 media (Adv. DMEM/F12 (Gibco, 12634010), 1 % (v/v) B27 (Gibco, 17504044), 1 % (v/v) L-Gln (Gibco, 25030-024) or GlutaMAX (Gibco, 35050061), 1 % (v/v) Pen/Strep (Gibco, 15070063), 2.5 µl EGF (100 µg/ml), PeproTech, 315-09; 2.5 µl FGF (100 µg/ml), PeproTech, 100-18B per 25 ml M 3 media).
6. After 72 hours, transfer the organoids to 6-well suspension plates (Greiner, 657185) and fill each well with up to 3 mL of M3 media.
7. Replace the M3 media at least every 72 hours for continued culturing.

### Culturing of aNSC-derived Cerebral Organoids (Utilizing a CERO 3D Incubator & Bioreactor)

*Overview:* Traditional culturing methods using an orbital shaker or 6-well plates can lead to flattened or oval organoids over time. By switching to the CERO 3D Incubator & Bioreactor, round and uniform organoids can be sustained for extended periods with minimal hands-on work. The larger culture volume in 50 mL CERO tubes reduces the frequency of interventions, making this method efficient and scalable.

### Procedure:

### Pre-seeding in ULA Plates:

1. Follow the neurosphere culture protocol (3.2) to prepare a single-cell suspension.
2. Determine cell count using a Neubauer chamber and Trypan Blue (Carl Roth, CN76.3) staining.
3. Seed 5,000-20,000 cells per well in a 96-well ULA plate (Nunclon, 174929) with 200 µL of M1 media (Adv. DMEM/F12, 1 %B27, 1 %L-Gln, 1 %GlutaMAX, 1 %N-2 supplement, 1 %Pen/Strep, 7.5 µl EGF (100 µg/ml, PeproTech, 315-09); 7.5 µl FGF (100 µg/ml), PeproTech, 100-18B) per 25 ml media). Transfer to CERO Tubes:
4. After 3 days, transfer organoids from the ULA plates to CERO tubes.
5. Add M2 media (minimum 12 mL per tube), containing Adv. DMEM/F12, 1 % B27, 1 % L-Gln, 1 % GlutaMAX, 1 % N-2 supplement, 1 % Pen/Strep, 5 µl EGF (100 µg/ml, PeproTech, 315-09) and 5 µl FGF (100 µg/ml, PeproTech, 100-18B) per 25 ml media.

### Media Change and Incubation:

6. Media changes are performed twice per week. Replace half of the media at each change or, if the media retains a healthy color (as indicated by phenol red), simply add 3 mL of fresh media.
7. After 1 week of incubation in M2, switch to M3 media (Adv. DMEM/F12, 1 % B27, 1 % L-Gln or GlutaMAX, 1 %Pen/Strep, 2.5 µl EGF (100 µg/ml), PeproTech, 315-09; 2.5 µl FGF (100 µg/ml), PeproTech, 100-18B per 25 ml M3 media for all subsequent media changes.
8. Organoids can grow over 2 mm in diameter within 3 months and prolonged cultivation for over 5 months is feasible under these conditions.

### Incubation Parameters:

For initial organoid growth, set the CERO bioreactor to rotate 1 second clockwise and 1 second counterclockwise with no break between rotations to maintain optimal mixing.

Once organoids reach a diameter of 250 µm, adjust the rotation protocol to the following: Rotate 2 seconds clockwise, pause for 1 second, then rotate 2 seconds counterclockwise, followed by another 1 second pause. These adjustments help maintain ideal culture conditions as the organoids grow larger.

### Alternative Method: Direct Neurosphere Outgrowth to Organoid Culture:

Another option to simplify cerebral organoid culture is to allow CERO-cultivated aNSCs to outgrow their neurosphere state. Once neurospheres reach a diameter of 100 µm or more, transition them to organoid culture as follows:

### Media Transition:

1. Replace the neurosphere media with M1 media once the neurospheres reach 100 µm in diameter.
2. After 1 week, switch to M2 media for the next media change.
3. After another week, perform all subsequent media changes using M3 media.

This method further reduces hands-on time, consumables and overall complexity, while enhancing the reproducibility of the organoid cultures.

**Table 3: Organoid media composition: A summary.**

| **M1** | **M2** | **M3** |
|---|---|---|
| Adv. DMEM/F12 | Adv. DMEM/F12 | Adv. DMEM/F12 |
| 1 % (v/v) B27 | 1 % (v/v) B27 | 1 % (v/v) B27 |
| 1 % (v/v) L-GlutaMAX | 1 % (v/v) L-GlutaMAX | 1 % (v/v) L-Gln or GlutaMAX |
| (Optional) 1 % (v/v) N-2 supplement | (Optional) 1 % (v/v) N-2 supplement | 1 % (v/v) Pen/Strep |
| 1 % (v/v) Pen/Strep | 1 % (v/v) Pen/Strep | 2.5 µl EGF (100 µg/ml) |
| 7.5 µl EGF (100 µg/ml) | 5 µl EGF (100 µg/ml) | 2.5 µl bFGF (100 µg/ml) |
| 7.5 µl bFGF (100 µg/ml) | 5 µl bFGF (100 µg/ml) | |

### Troubleshooting (6-Well Suspension Plate on Orbital Shaker) vs. CERO Bioreactor

1. *Inadequate organoid growth:*
   ∘ Cause: Infrequent media changes can lead to nutrient depletion, stunting organoid growth.
   ∘ Solution (Orbital Shaker): Use media tailored to the current culture stage and change the media every 2-3 days to ensure a consistent nutrient supply.
   ∘ Solution (CERO): The CERO system contains a larger media reservoir within the CERO tubes, making frequent media changes unnecessary. This ensures a steady nutrient supply over time without constant intervention.
2. *Organoid clumping:*
   o Cause: A high number of organoids in a small reservoir can lead to clumping, hindering proper growth and development.
   ∘ Solution (Orbital Shaker): Reduce shaking intensity or manually separate the organoids. If clumping persists, transfer organoids to larger dishes to avoid overcrowding.
   ∘ Solution (CERO): The CERO bioreactor minimizes organoid clumping by providing constant, gentle rotation that allows organoids to remain suspended and evenly spaced, effectively bypassing this issue.
3. *Disruption during media changes:*
   o Cause: Organoids can be damaged or disrupted during media aspiration in traditional wells, leading to inconsistent results.
   ∘ Solution (Orbital Shaker): Carefully aspirate media from the side of the well to minimize disturbance and prevent damage.
   o Solution (CERO): In the CERO tubes, organoids naturally fall to the bottom due to gravity during media changes, allowing for straightforward aspiration without any risk of damaging the organoids.
4. *Uneven organoid size:*
   ∘ Cause: Uneven growth can result from inconsistent media exposure or variations in cell seeding density, leading to size discrepancies.
   ∘ Solution (Orbital Shaker): Ensure even cell distribution during seeding and maintain consistent incubation and shaking conditions. Monitor organoids regularly and transfer them to larger wells when necessary.
   ∘ Solution (CERO): The CERO bioreactor ensures even organoid growth by preventing clumping and allowing all organoids in the tube equal access to nutrients. The continuous rotation facilitates uniform growth conditions, ensuring more consistent results.
5. *Low cell viability in organoid cores:*
   o Cause: Organoids cultured for extended periods (over 3 months) can develop necrotic cores due to insufficient nutrient diffusion, especially in larger organoids.
   ∘ Solution (Orbital Shaker): Avoid overgrowth by limiting culture duration to 3 months. Regularly monitor size and change media to promote adequate nutrient supply.
   ∘ Solution (CERO): The CERO bioreactor's advanced mixing technology keeps organoids constantly suspended in well-mixed media. This prevents them from settling on the bottom, ensuring optimal nutrient distribution even to the cores of large organoids, thereby reducing the risk of necrosis or hypoxia.

### 9. Cancer core and spheroid core model

This protocol describes the step-by-step generation of Cancer Core and Spheroid Core models, integrating cancer cells (see Figure 3) or pre-formed spheroids (see Figure 4) with differentiated cerebral organoids.

### Cancer Core Model

The Cancer Core Model integrates single cancer cells into pre-differentiated cerebral organoids to simulate controlled tumor growth. This model generates distinct patterns depending on the cancer-to-cerebral cell ratio.

### Prepare cancer cell suspensions:

1. Detach and prepare single-cell suspensions from cancer cell lines (e.g., GL261-GFP, 99LN-BFP, LLC1-GFP, MK1735-BFP) following standard passaging protocols.
2. After the final centrifugation step, discard the supernatant and resuspend the cells in 2-5 mL of M3 media (Advanced DMEM/F12 (Gibco, 12634010), 1 % (v/v) Pen/Strep (Gibco, 15070063), 1 % GlutaMAX (Gibco, 35050061), 1 % B27 (Gibco, 17504044), 2.5 µl EGF (100 µg/ml), PeproTech, 315-09) , 2.5 µl FGF (100 µg/ml), PeproTech, 100-18B per 25 ml M3 media).
3. Filter the suspension through a 100 µm EASYstrainer (Miltenyi Biotec, 130-098-463) to ensure a uniform single-cell suspension.
4. Count the cells using a Neubauer chamber (Hycor, 87144) and assess viability using Trypan Blue (Carl Roth, CN76.3).

### Dissociation of cerebral organoids:

5. Transfer 50-100 pre-differentiated cerebral organoids (grown for a minimum of 1 month, diameter >300 µm) into a 15 mL Falcon tube. 6. Centrifuge the tube at 200 g for 2 minutes and discard the supernatant.
7. Add 1-2 mL of 0.05% trypsin-EDTA (Gibco, 25300054) to the organoids and incubate at 37 °C on an orbital shaker (Thermo Fisher, 88881101) for 2 minutes.
8. Triturate the organoids gently using a p1000 pipette, followed by a 200 µL pipette, until a uniform single-cell suspension is obtained.
9. Add 1 mL of trypsin inhibitor (Sigma-Aldrich, T6522) to stop the enzymatic reaction.
10. Centrifuge again at 200 g for 2 minutes and discard the supernatant.
11. Resuspend the pellet in 1-2 mL of M3 media and filter through a 100 µm EASYstrainer.
12. Count the dissociated organoid cells using a Neubauer chamber.

### Assemble the Cancer Core model:

13. Prepare the desired cerebral organoid-to-cancer cell ratios, typically ranging from 2:1 to 20:1 (cerebral organoid cells to cancer cells).
14. Seed 200 µL of the cell mixture into each well of a U-shaped 96-well ULA late (Nunclon, 174929).
15. Incubate the plates at 37 °C in a humidified 5% CO₂ incubator on an orbital shaker for 72 hours to facilitate the formation of the cancer core model.

### Spheroid Core Model

The Spheroid Core Model embeds pre-formed cancer spheroids into cerebral organoids to mirror TMEs, ideal for medium-throughput drug efficacy and neurotoxicity screening.

### Generate cancer spheroids:

1. Prepare a single-cell suspension from cancer cell lines as outlined in Step 1 of the Cancer Core Model.
2. After counting and viability assessment, resuspend the cells in spheroid-specific media (DMEM (Gibco, 21969-035) supplemented with 10% FBS (PAN-Biotech, P30-3302), 1 %L-Gln (Gibco, 25030-024), 1 %Pen/Strep (Gibco, 15070063) and optionally 12.5 ng/mL EGF (PeproTech, 315-09)).
3. Seed 10,000-40,000 cells per well into U-shaped 96-well ULA plates (Nunclon, 174929) in 200 µL of media.
4. Incubate at 37°C in a humidified 5% CO₂ incubator for 48-72 hours to allow for spheroid formation.

### Dissociate cerebral organoids for spheroid core formation:

5. Follow Step 2 of the Cancer Core Model to enzymatically dissociate pre-differentiated cerebral organoids into a uniform single-cell suspension.

### Assemble the Spheroid Core model:

6. Combine 100,000 dissociated cerebral cells with one pre-formed cancer spheroid per well in 200 µL of M3 media.
7. Seed the mixture into a U-shaped 96-well ULA plate.
8. Incubate at 37 °C in a humidified 5% CO₂ incubator on an orbital shaker for 72 hours to promote integration and formation of the spheroid core model.

### Troubleshooting

*1. Cell Adherence to Ultra-Low Attachment Plates*
   o Cause: Despite the design of ultra-low attachment plates, cells may still adhere to the surface.
   o Solution: Pre-treat the plates with an anti-adherence solution and incubate them overnight before seeding to prevent unwanted cell attachment.
*2. Imbalanced Healthy-to-Cancer Cell Ratio*
   o Cause: An incorrect ratio of healthy to cancer cells can negatively impact the formation of the cancer core model.
   o Solution: Experimentally optimize the cell ratio for each cell line to ensure balanced model development and consistent results.
*3. Disaggregation of the Cancer Core Model*
   o Cause: The 3D cancer core may disaggregate if cells fail to form stable connections during the early stages of the model.
   o Solution: Ensure sufficient cell density to promote robust cell-cell adhesion and allow ample incubation time for interactions to stabilize before applying mechanical agitation.
*4. Clumping During Single-Cell Preparation*
   o Cause: Tumor cells may form clumps after trypsinization, making it difficult to achieve an accurate cell count.
   o Solution: If single-cell suspensions are not prepared in parallel, keep the tumor cells on ice immediately after trypsinization to minimize clumping and maintain a uniform single-cell suspension.

### 10. Advanced cancer core and advanced spheroid core model

This protocol describes the formation of the Advanced Cancer Core Model, integrating cerebral organoid cells, cancer cells, and microglia to simulate tumor-immune interactions (see Figure 5).

### Advanced Cancer Core Model

### Cerebral Organoid Dissociation:

1. Transfer 50-100 pre-differentiated cerebral organoids (grown ≥1 month, diameter >300 µm) into a 15 mL Falcon tube (Greiner Bio-One, 188271). Centrifuge at 200 g for 2 minutes.
2. Aspirate the supernatant and resuspend the organoids in 1-2 mL of 0.05% trypsin-EDTA (Gibco, 25300054). Incubate at 37°C on an orbital shaker (Thermo Fisher Scientific, 88881101) for 2 minutes.
3. Triturate the organoids with a p1000 pipette (Eppendorf, 3120000946), then switch to a p200 pipette until a uniform single-cell suspension is obtained. 4. Stop the enzymatic reaction by adding trypsin inhibitor (Sigma-Aldrich, T6522). Centrifuge again at 200 g for 2 minutes, discard the supernatant, and resuspend in M3 media (Advanced DMEM/F12 (Gibco, 12634010), 1% (v/v) B27 (Gibco, 17504044), 1% L-Glutamine (Gibco, 25030-024), 1% GlutaMAX (Gibco, 35050061), 1% Pen/Strep (Gibco, 15070063), 10 ng/mL EGF (PeproTech, 315-09), 10 ng/mL bFGF (PeproTech, 100-18B), 50 µL IL-34 (100 µg/mL), 2.5 µL TGF-β (50 µg/mL) per 25 mL).

### Prepare cancer cell suspensions:

5. Detach and prepare single-cell suspensions from cancer cell lines (e.g., GL261-GFP, 99LN-BFP) following standard passaging protocols.
6. After the final centrifugation step, discard the supernatant and resuspend the cells in 2-5 mL of M3 media (Advanced DMEM/F12 (Gibco, 12634010), 1 % (v/v) Pen/Strep (Gibco, 15070063), 1 % GlutaMAX (Gibco, 35050061), 2 % (v/v) B27 (Gibco, 17504044), 2.5 µl EGF (100 µg/ml, PeproTech, 315-09), 2.5 µl FGF (100 µg/ml, PeproTech, 100-18B) per 25 ml M3 media).
7. Filter the suspension through a 100 µm EASYstrainer (Miltenyi Biotec, 130-098-463) to ensure a uniform single-cell suspension.
8. Count the cells using a Neubauer chamber (Hycor, 87144) and assess viability using Trypan Blue (Carl Roth, CN76.3).

### Microglia Preparation:

9. Culture microglia (e.g., EOC2 cells) in DMEM compl. media (DMEM (Gibco, 11995065), 10% FBS (PAN-Biotech, P30-3302), 1% L-Glutamine (Gibco, 25030-024), 1% Pen/Strep (Gibco, 15070063)) supplemented with IL-34 (50 µL, 100 µg/mL) and TGF-β (2.5 µL, 50 µg/mL). Detach the cells using a cell scraper (Corning, 3010), count them with a Neubauer chamber, and assess viability with Trypan Blue.

### Mastermix Preparation:

10. Prepare a mastermix with desired ratios of cerebral organoid cells, cancer cells, and microglia (e.g., 2:1:1, 4:1:2). For example, for a 2:1:1 ratio, use 20,000 cerebral organoid cells, 10,000 cancer cells, and 10,000 microglia per well.
11. Add 200 µL of the mastermix to each well of a U-bottom 96-well ULA plate (Nunclon, 174929).

### Incubation and Shaking:

10. Place the plates on an orbital shaker inside a 37°C, 5% CO₂ incubator and shake the plates at 100 rpm for 72 hours to promote Advanced Cancer Core formation.

### Advanced Spheroid Core Model

The Advanced Spheroid Core Model integrates pre-formed cancer spheroids, dissociated cerebral organoids, and microglia to recreate a tumor microenvironment for studying tumor-immune interactions and brain tumor progression (see Figure 6).

### Advanced Spheroid Core Model

### Cancer Spheroid Generation:

1. Prepare a single-cell suspension from cancer cells (e.g., GL261 or 99LN) and seed 10,000 to 40,000 cells per well into U-bottom 96-well ULA plates (Nunclon, 174929).
2. Add 200 µL of spheroid-specific media (DMEM (Gibco, 21969-035), 10% FBS (PAN-Biotech, P30-3302), 1% L-Glutamine (Gibco, 25030-024), 1% Pen/Strep (Gibco, 15070063)) per well.
3. Incubate the plates at 37°C in a 5% CO₂ incubator for 48-72 hours to allow spheroid formation.

### Cerebral Organoid Dissociation:

4. Transfer 50-100 cerebral organoids (>300 µm) into a 15 mL Falcon tube (Greiner Bio-One, 188271) and centrifuge at 200 g for 2 minutes.
5. Resuspend the organoids in 1-2 mL of 0.05% trypsin-EDTA (Gibco, 25300054). Incubate on an orbital shaker at 37°C for 2 minutes.
6. Triturate with a p1000 pipette (Eppendorf, 3120000946) and a p200 pipette until a uniform single-cell suspension is obtained.
7. Stop the enzymatic reaction with trypsin inhibitor (Sigma-Aldrich, T6522). Centrifuge at 200 g for 2 minutes, discard the supernatant, and resuspend in M3 media.

### Microglia Preparation:

8. Culture microglia (e.g., EOC2 cells) in DMEM compl. media (DMEM (Gibco, 11995065), 10% FBS (PAN-Biotech, P30-3302), 1% L-Glutamine (Gibco, 25030-024), 1% Pen/Strep (Gibco, 15070063)) supplemented with IL-34 (50 µL, 100 µg/mL) and TGF-β (2.5 µL, 50 µg/mL). Detach the cells using a cell scraper (Corning, 3010), count them with a Neubauer chamber, and assess viability with Trypan Blue.

### Advanced Spheroid Core Assembly:

9. Combine 100,000 dissociated cerebral organoid cells, one pre-formed cancer spheroid, and 10,000-40,000 microglia per well in 200 µL of M3 media. Seed the mixture into each well of a U-bottom 96-well ULA plate.
10. Incubate at 37°C in a humidified 5% CO₂ incubator for 24 hours without shaking to allow for initial cell interactions and spheroid stabilization.

### Incubation and Shaking:

11. After 24 hours, transfer the plates to an orbital shaker inside the incubator and shake at 100 rpm for 48 hours to facilitate the formation of the Advanced Spheroid Core model.

### Troubleshooting

*1. Cell Adherence to Ultra-Low Attachment Plates:*
   o Cause: Despite the ULA plate design, cells may still adhere to the surface.
   o Solution: Pre-treat plates with an anti-adherence solution (e.g., Pluronic F-127) and incubate overnight to prevent unwanted cell attachment.
*2. Imbalanced Cell Ratios:*
   o Cause: Incorrect cerebral organoid-to-cancer cell ratio can impact model formation.
   o Solution: Optimize cell ratios experimentally for each cell line to ensure balanced core formation and reproducibility.
*3. Disaggregation of Models:*
   o Cause: Models may disaggregate if cells fail to form stable connections early on.
   o Solution: Ensure sufficient cell density and incubation time to promote robust cell-cell adhesion before applying mechanical agitation.
*4. Clumping During Single-Cell Preparation:*
   o Cause: Tumor cells may clump after trypsinization, leading to uneven cell distribution.
   o Solution: Keep cells on ice immediately after trypsinization to prevent clumping and ensure a uniform single-cell suspension.
5. *Low Microglia Viability:*
   o Cause: Microglia may exhibit reduced viability during the model formation process.
   o Solution: Supplement media with IL-34 and TGF-β to support microglia function and maintain their viability throughout the process.

### B. Results

### 1. New tumor assembloid models

Herein, the inventors introduce a pioneering platform that adheres to the 3R principles using adult murine neural stem cells (aNSCs). This innovation circumvents the resource-heavy requirements of inducing iPSCs, presenting a suite of both 2D and 3D models tailored for robust early-stage drug assessments (Fig. 7a).

These models, ranging from *in vitro* to *ex vivo* systems, offer increasing biological complexity for precise analysis of cell-type-specific drug effects (Fig. 7b, c). The inventors developed 2D models using undifferentiated and differentiated neural cells and 3D cerebral organoids for high- and medium-throughput neurotoxicity screenings, respectively. Complementing these are three 3D models combining cerebral organoids with primary and secondary brain cancer cells, forming assembloids that closely mimic tumor microenvironment (TME) interactions. The standout 3D models include: 1) The *Assembled Model,* fusing pre-grown cancer spheroids with organoids to study cell migration; 2) The *Spheroid Core* and *Cancer Core Models,* where cancer spheroids or single cancer cells are embedded within cerebral organoids to simulate distinct TMEs; 3) The *Advanced Spheroid Core* and *Advanced Cancer Models,* which further incorporate microglia, enriching model complexity to simulate immune interactions.

These assembloid platforms enable simultaneous medium-throughput drug efficacy and neurotoxicity assessments. Importantly, their composition - tumor morphology or infiltration patterns - closely mirrors clinical conditions, making them valuable for studying solid brain cancers. The models facilitate a comprehensive evaluation of drugs through a range of progressively intricate systems, offering robust early-stage screening for both therapeutic efficacy and neurotoxicity. By integrating early neurotoxicity testing, our platform bridges the gap between traditional *in vitro* and *in vivo* studies.

Crucially, for the first time, the *Cancer Core Model* allows for the reproducible seeding of defined cancer and non-cancerous cell numbers within 3D assembloids, enabling controlled tumor growth inside the organoids. This innovation also allows the inclusion of a specified number of microglia throughout the model, termed the *Advanced Cancer Core.* This model can replicate TMEs with microgliosis, as observed in breast-to-brain metastasis, or glioma-rich tumor masses, reflecting the clinical scenarios of high-grade gliomas.

This suite of models, designed in line with the European 3R directive, represents a significant ethical and methodological advancement in brain cancer research. It promises not only improved avenues for treatment development but also a commitment to responsible scientific innovation.

These models, spanning *in vitro* to *ex vivo* systems, offer escalating biological complexity for precise analysis of cell-type-specific drug impacts (Fig. 7b,c). In detail, the inventors created models based on 2D healthy un-/differentiated neural brain cells and 3D differentiated neural brain cells for high- and medium-throughput neurotoxicity screening, respectively. Furthermore, the inventors accessed three 3D models that combine differentiated cerebral organoids with various primary and secondary brain cancer cells - creating assembloids that facilitate cell-cell cross-communication as seen in the tumor microenvironment. The inventors' standout 3D models include 1) The assembled model, fusing pre-grown cancer spheroids with organoids to study cell migration; 2) The spheroid core and cancer core models, where cancer spheroids or single cancer cells are embedded within cerebral organoids to simulate distinct tumor architectures; 3) The advanced cancer core model, which further incorporate immune cells such as microglia, enriching model complexity to simulate the immune compartment. All assembloid models serve as a medium-throughput platform for drug efficacy and neurotoxicity assessment. Notably, model composition, such as tumor morphology and infiltration, depends strongly on the cancer cell lines used and closely resembles the clinical situation. This suite of models facilitates comprehensive drug evaluation through a spectrum of intricately refined models, each increasing in complexity. Implemented as an early drug-discovery testing platform, it is set to revolutionize anti-brain cancer drug testing by incorporating early neurotoxicity screening, thereby effectively closing the translational gap between *in vitro* and *in vivo.* Embodying the European 3Rs directive, it marks a significant ethical and methodological advancement in brain cancer research, promising not just improved treatment avenues but also a commitment to responsible scientific practices.

### 2. Generation and characterization of murine adult neural stem cell culture

To identify stem-cell-rich regions within the murine brain, the inventors harvested brains from C57BL/6 young adult mice aged nine to 20 weeks. After fixation, the inventors sectioned the brains using a vibratome (Vibratome Leica vt1200s, 1491200S001, Leica Microsystems Vertrieb GmbH) and cleared 300 µM thick sections, subsequently staining them with stem-cell markers Nestin (MAB353, Sigma-Aldrich) and SOX-2 (AF2018, R&D Systems), neural marker NeuN (ab177487, Abcam), and nuclear stain DAPI (D9542, Sigma-Aldrich). The inventors identified enriched murine neural stem cell colonization in the lateral ventricle walls of the subventricular zone and the dentate gyrus of the subgranular zone (Fig. 8a, c). Following a previously published and modified protocol40,41, the inventors isolated respective areas from freshly dissected murine brains, processing them via mechanical dissection and enzymatic digestion using trypsin (trypsin-EDTA (0.05 %), 25300054, Gibco), followed by trypsin inhibition (trypsin Inhibitor from Glycine max (soybean), T6522, Sigma-Aldrich) and filtering through a 100 µm cell strainer to collect a single-cell suspension. This suspension was then cultivated in neural stem cell basal media (Neural Stem Cell Basal Medium, SCM003, Sigma-Aldrich) (Material and methods, A.2 and A.7). Over one to two weeks, cellular debris diminished, and well-shaped neurosphere cultures emerged. By day 14, these proliferating cell aggregations reached diameters of 50-100 µm and were primarily composed of stem cells and progenitor cells (Fig. 8d).

To assess aNSCs-based neurospheres for stem cell characteristics and differentiation potential, the inventors seeded 5,000 cells per well into poly-L-ornithine (P4957, Sigma-Aldrich) and laminin (L2020, Sigma-Aldrich)-coated 96-well plates (96-well clear flat bottom polystyrene TC-treated plate, 3596, Corning), according to a previously published and modified protocol40 (Fig. 8b). The inventors generated a fresh single-cell suspension by quickly digesting cultivated neurospheres with trypsin, followed by trypsin inhibition and cell counting using a hemocytometer. For evaluating stem cell characteristics, cells were allowed to attach for 24 hours before being fixed with formaldehyde (Roti^{®}-Histofix 4 %, 2213.3, Carl Roth) and stained for neural stem cell marker SOX-2, along with differentiation markers NeuN, Olig2 (ab109186, Abcam), and GFAP (ab53554, Abcam) for neurons, oligodendrocytes, and astrocytes, respectively. Conversely, to analyze 2-dimensional (2D) differentiation potential, seeded cells were incubated in growth-factor-reduced media for 5 days before fixation and staining. Confocal immunofluorescence (IF) images of undifferentiated aNSCs showed high abundance of the stem cell marker SOX-2, indicating robust stem cell characteristics (Fig. 8e). In contrast, aNSCs incubated with growth factor withdrawal exhibited a loss of SOX-2 expression but showed a high abundance of Olig-2, and GFAP confirming the presence of oligodendrocytes, and astrocytes respectively (Fig. 8f). Furthermore, the neural morphology exhibits a highly interconnected network indicative of advanced neural differentiation. Therefore, aNSC-based neurospheres are capable of effectively generating the primary cell types of brain parenchyma.

### 3. aNSCs-based assay for 2D high-throughput neurotoxicity assessment

With proven high stem cell potency and efficient maturation via growth-factor starvation, the inventors explored the potential of aNSCs-based 2D models as novel high-throughput neurotoxicity assays. The inventors aimed to establish a straightforward and effective neurotoxicity screening method tailored for both undifferentiated and differentiated neural stem cells. The inventors selected the tetrazolium compound [3-(4,5-dimethylthiazol-2-yl)-5-(3-carboxymethoxyphenyl)-2-(4-sulfophenyl)-2H-tetrazolium] (MTS)-based assay (CellTiter 96^{®} AQueous One Solution Cell Proliferation Assay, G3582, Promega) as quantitative, colorimetric, and microplate reader-compatible standard for cell proliferation. Mechanistically, the tetrazolium compound MTS is bio-reduced by metabolically active cells giving rise to a violet-colored formazan product. The resulting formazan's absorbance at 490 nm directly correlates with the number of viable cells, making this single-step reagent addition ideal for homogeneous high-throughput screenings.

In the inventors' proof-of-concept experiment, they seeded 6,000 aNSCs per well into pre-coated 96-well plates. The cells were allowed to attach for 12 hours to assess the effects of drugs on cells with high stem cell characteristics (Fig. 9a) or to attach and differentiate for 3 days for neurotoxicity assessment in neurons, astrocytes, and oligodendrocytes (Fig. 9b). As a reference, the standard murine glioma cell line GL261 was included in the experiment (Fig. 9c), with cancer cells being allowed to attach for 12 hours prior to drug exposure. The inventors then treated the adherent aNSCs and GL261 cells with the U.S. Food and Drug Administration (FDA)-approved, standard-of-care chemotherapeutic agent Temozolomide (TMZ, the agent of choice in glioblastoma treatment) at a therapeutic dose of 500 µM for 48 hours, followed by the application of fresh media to simulate a 48-hour recovery period post-TMZ treatment.

Cell viability was quantified using MTS assays at 3, 6, 12, and 24 hours post-TMZ treatment, as well as at 48 hours post-treatment and after an additional 24 or 48-hour recovery period. The assays revealed significant differences in drug susceptibility among the stem cell-based, differentiated cell-based, and GL261 cell-based models. As an alkylating agent, TMZ induces DNA damage and thereby inhibits cell growth, with fast-cycling cells being particularly affected. In undifferentiated aNSCs, which are fast-growing, the impact of TMZ was pronounced, with a 50% loss in cell viability observed after 48 hours of treatment compared to the control at this time point. In contrast, differentiated aNSCs treated with TMZ maintained 80% cell viability at the same time point. Consistent with previous findings, TMZ only slightly reduced viability in the glioma cell line GL261. Unlike aNSCs, TMZ-treated GL261 cells rapidly recovered during the 48-hour recovery period, underscoring the aggressive nature of glioma cells. In summary, this proof-of-concept model clearly highlights aNSC models as a rapid and effective system for screening neurotoxicity versus efficacy. This simple setup provides a straightforward in vitro method for analyzing drug-dependent neurotoxicity, offering valuable insights into drug toxicity in non-malignant cells.

### 4. aNSCs 3D differentiation to access mature cerebral organoids

Leveraging the inherent high stem cell character of aNSCs, the inventors aimed to generate differentiated 3D cerebral organoid cultures. Utilizing a modified protocol, the inventors seeded 10,000 aNSCs per well into ultra-low adherent (ULA) 96-well plates (Nunclon^{™} Sphera^{™} Microplates, Thermo Scientific, 174929) to promote the formation of well-defined spherical organoids. For differentiation, the inventors incubated these 3D cultures in media laden with pre-defined differentiation factors (see Material and methods, A.3, A.8), ensuring a conducive environment for organoid growth. To facilitate optimal growth and even distribution of differentiation factors, the inventors placed the plates on a CO₂-resistant orbital shaker (88881101, Thermo Fisher Scientific Inc.), maintained at 37°C with 5% CO₂.

Once the organoids reached diameters exceeding 300 µm, the inventors carefully transferred them using single-use 3 ml Pasteur pipettes to 6-well suspension plates (Brand, Model #), each containing 3 ml of differentiation media III. Transferred to the orbital shaker, the cerebral organoids were gently swirled in circular motions, promoting their growth into well-defined spheres (Fig. 10a, c). This methodological approach not only facilitates the growth of cerebral organoids but also ensures their uniform development and maturation, offering a robust platform for neurological studies and applications.

### 5. Large Organoid Preparation and Analysis

To access large cerebral organoids exceeding 2 mm in diameter, the inventors extended the culture period to three months before fixing them. The inventors employed fixation techniques optimized for large in-well processing and agarose embedding, facilitating subsequent paraffin embedding and sectioning. These organoids underwent immunohistochemical (IHC) (Fig. 10d) and IF staining (Fig. 10b, c, e and f). Hematoxylin and eosin (H&E) staining revealed differentiated cellular zones toward the organoid's periphery and well-defined structures within, indicative of the advanced maturation observed over the growth period. Additionally, cleaved Caspase-3 and Ki-67 staining showed minimal apoptosis and high proliferation, respectively, particularly around the organoid's outer rim. Using antibodies against GFAP, Olig2, and TUBB3, the inventors identified distinct spatial distribution patterns within the organoids. Neuronal populations predominantly localized at the periphery, reflecting the organized growth patterns aimed for with our aNSC-based approach, facilitated by our CO₂-resistant orbital shaker system. In contrast, glial cells, including astrocytes and oligodendrocytes, were more centrally located. These findings underscore the robustness and viability of the cerebral organoids cultivated in our modified system and the efficacy of the dynamic environment provided by the orbital shaker for even distribution of differentiation factors.

### 6. Extension of 3D differentiation protocol for assembloid models

To generate models recapitulating non-cancerous and cancerous tissue within the TME, the inventors next established fused assembloid models for primary and secondary brain cancers. To this end, the inventors employed two distinct methodologies: the 'assembled' and the 'cancer core' model. For the 'assembled' model, organoids cultured to diameters of 0.8-1 mm were co-cultured with previously seeded spheroids. Depending on the specific cancer cell line, the inventors seeded between 1,000 to 5,000 cancer cells into ULA plates to form a spheric 3D culture. The 3D growth of these cultures was monitored using BF and fluorescence microscopy, for cell lines carrying reporter genes like green, red, or blue fluorescent protein (GFP, RFP, or BFP). After spheroid formation (period of 48-72 hours), cancer spheroids were combined with cerebral organoids that had undergone a minimum of four weeks of growth and differentiation. This process involved transferring single cerebral organoids into fresh ULA wells, and co-culture with a previously grown cancer spheroid of choice.

In this proof-of-concept study, the inventors utilized three cell lines including GL261 and its reporter gene expressing GL261-GFP, the breast-to-brain cell line 99LN and 99LN-BFP or 99LN-GFP, as well as the melanoma-to-brain cell line MK1735 (MK) and MK-BFP. These models were cultured in media M3. To establish proximity, the plates containing one organoid and one spheroid per well were briefly centrifuged and then incubated at 37°C with 5% CO₂. After the initial 12-hour cell attachment phase, the ULA plates were placed on a shaker to ensure enhanced nutrient distribution within the media, thus minimizing well effects. Respective models were monitored via BF and fluorescence (EVOS, X, Invitrogen^{™}). For structural analysis, the assembled models were fixed and prepared for IHC and IF studies. H&E staining showed the formation of stable cell-cell interactions between cerebral organoids and cancer spheroids. Remarkably, 99LN and MK spheroids fully integrated into the cerebral organoid giving rise to models with defined cancerous tissue fully surrounded by non-cancerous tissue. Additionally, cleaved Caspase-3 and Ki-67 staining indicate minimal apoptosis and high proliferation, respectively, especially in the cancer spheroids and around the organoids' outer rim.

### 7. Real-Time Microscopy Analysis of Interactions Between Cerebral Organoids and Cancer Spheroids

To visualize the dynamic interactions and integration patterns within our 'assembled' model, the inventors employed the CellVoyager^{™} CQ1 Benchtop High-Content Analysis System (Yokogawa) for live cell imaging over 72 hours. The inventors documented the formation of tight cell-cell interactions between cerebral organoids and cancer spheroids, noting substantial engagement as early as 12 hours post-co-culture, with progressive intensification observed over time (Fig. 11a). Notably, by the 24-hour mark, the inventors recorded the direct integration of cancer cells within the cerebral organoid, underscoring the reciprocal interplay between oncogenic and brain cells (Fig. 11b and c). Contrary to prevailing expectations, the cerebral organoid demonstrated an active adaptation to the infiltrating cancer cells, accommodating and, in some cases, entirely engulfing the cancer spheroid. This behavior mimics the characteristics of solid tumors with limited invasiveness seen in the brain's parenchymal tissue, thereby enhancing the model's relevance to clinical conditions. Given the aggressive and invasive nature of certain cancers like glioblastoma, characterized by their diffusive and undefined boundaries in brain cancer patients, the inventors recognized the need for a more representative model.

### 8. Extension of co-culture protocol for infiltrative 'cancer core' models

To develop sophisticated models that accurately represent the dynamic interactions within the tumor microenvironment (TME), the inventors refined their co-culture protocol to give rise to the 'cancer core' model. To this end, primary (GL261, GL261-GFP) or secondary cancer cell lines (99LN, 99LN-BFP, 99LN-GFP) were again carefully prepared through a series of steps to give rise to single-cell suspensions: medium removal, washing with PBS, trypsinization for detachment, neutralization with DMEM complete, and subsequent centrifugation and resuspension. Next, contrary to the above-described methodology for 'assembled' models, cerebral organoids were also processed through centrifugation, trypsinization, trituration, and resuspension, resulting in a single-cell suspension for co-cultivation. Following the preparatory steps, the cancer cells and cerebral organoids were seeded at precise ratios into ultra-low attachment (ULA) plates, a technique inspired by the inventors' 'assembled' model where spheroids and organoids are brought together. The co-culture was allowed to form without centrifugation to allow the formation of different interaction patterns depending on the respective cell ratios. The cultures were incubated at 37°C and 5% CO₂ on an orbital shaker (100 rpm). Monitoring of the co-culture was conducted using bench-top fluorescence microscopy for real-time visualization of interactions (Fig. 12a). As a control, single-cell suspensions of cerebral organoids were reseeded at 100,000 cells per well. 3D formation was monitored using a benchtop fluorescence microscope (Fig. 12b). Post-seeding, the assembled models were allowed for 80 hours to reform and stabilize into solid, well-structured entities. The inventors then prepared their models for IHC and IF studies, to visualize cell morphology, proliferation, and distribution of various cell types. Depending on the chosen cancer cell lines and cell ratios, the inventors observed models with well-defined cancer cores or with highly invasive, diffused cancer entities throughout the cerebral organoid. As a rule of thumb, 1:100 to 5:100 cancerous cells to non-cancerous cells resulted in infiltrative patterns, while ratios from 1:10 exhibited more solid tumor cores. The rationale behind this may be the fact that cancerous cells are heavier and thus fall quicker to the bottom of the well, resulting in a core aggregation of cancerous cells if a sufficient number of cancer cells is available in the co-culture. IHC stainings showed highly proliferating cancer cores as well as infiltrative cancer cells distributed throughout the model. The non-cancerous tissue exhibited discrete areas with high proliferation (Ki67 staining) and minimal apoptosis (cl. Cas-3 staining) (Fig. 12c). Additionally, IHC staining of the reseeded cerebral organoid control revealed distinct 3D spheres (H&E) with strong proliferation (Ki67) and minimal apoptosis (Caspase-3) (Fig. 12d). IF staining showed a high abundance of NeuN or MAP2, Olig2, and GFAP, alongside cell morphology associated with differentiated neurons, oligodendrocytes, and astrocytes, therefore confirming cell maturity. Thus, the inventors' 'cancer core' model overcomes limitations of the 'assembled' model by replicating a TME, characterized by their diffusive and undefined boundaries as seen in the clinic.

### 9. Integrating microglia to enhance tumor microenvironment modeling

To enhance the complexity of the inventors' cancer core model and accurately replicate the immune component within the TME, the inventors incorporated microglia (EOC2) into the 'cancer core' to give rise to the 'advanced cancer core' model. This process followed the previously described protocol to generate single-cell suspensions of cerebral organoids, the respective cancer cell line, and this time, also microglia cells. In a proof-of-concept experiment, cerebral organoids, GL261-GFP, and 99LN-BFP, along with EOC2, were prepared as stock solutions. Both cerebral organoids and cancer cells underwent the above-described trypsin digest to result in single-cell solutions. Contrary, EOC2 underwent mechanical dissociation from the culture flask bottom and were filtered. Next, the respective single-cell stock solutions were plated together in an ULA 96-well plate. The plating ratios for non-cancerous brain cells, cancer cells, and microglia EOC2 were 2:1:1, 4:1:1, and 4:1:2, respectively. To provide a comparative control, the inventors also seeded respective non-cancerous to cancerous ratios without EOCs. Importantly, co-cultures with EOC2 were grown in optimized media containing TGF-β and Interleukin 34 (IL-34) in the mix. The respective plates were then placed on an orbital shaker set at 37°C and 5 % CO₂, operating at 100 rpm. Fluorescence microscopy was employed to monitor the co-cultures. Remarkably, models containing EOC2 exhibited significant differences in cell distribution after 24 hours displaying a mosaic-like cell distribution, compared to EOC2-free controls. However, this effect diminished over time, as detailed in Figure 13. At 72 hours post-seeding, these well-structured entities were fixed and prepared for IHC (Fig. 13) and IF analysis to further elucidate the intricate interactions and structural integrity.

The integration of microglia resulted in a more complex tumor microenvironment (TME). Notably, EOC2 was visualized using Anti-TMEM 119 (400 004, SYSY) in IHC. The advanced core models closely resemble clinical histology, with GL261-GFP glioma cores displaying high EOC2 content within the infiltrative tumor mass (Fig. 13, top right), and the exclusion of microglia observed in breast-to-brain metastasis, as replicated in the inventors' advanced 99LN-BFP breast-to-brain model (Fig. 13, bottom right). This enables the utilization of the 'advanced cancer core' model for advanced applications such as immunotherapy screening and complex multimodal treatment assessments. This development paves the way for highly modulable and individualizable models, setting a new standard for rational drug screening strategies. With these enhancements, the approach of the invention offers a versatile platform for exploring a wide range of therapeutic interventions, tailored to the specific needs and characteristics of the TME.

### 10. Employing Core Cancer Assembly Models for Medium-Throughput Screening to Investigate Cell-Type Dependent Drug Effects

To address the deficiency of complex brain tumor microenvironment (TME) models for early drug neurotoxicity and efficacy screenings, the inventors employed their novel 'cancer core' model in a medium-throughput assay.

In this proof-of-concept experiment, Act-B RFP-labeled cerebral organoids and GFP-labeled GL261 glioma cells were utilized to establish a 'glioma core' model tailored for drug screening. The inventors treated the assembled 'glioma core' models with selected agents for a 24-hour duration, using drug concentrations derived from prior 3D tumor spheroid screenings. Specifically, initial screenings were conducted with drug concentrations between IC20-IC25, applying 2 µM of the FDA-approved drug Auranofin^{®} (AUF) dissolved in 1 % (v/v) DMSO or a vehicle control (1 % (v/v) DMSO) for 24 hours. To mitigate technical and biological variability, each experimental condition was replicated nine times.

The integrity of the 3D models was monitored using BF and fluorescence microscopy, focusing on structural changes within the 'glioma core' model. Before treatment, all models exhibited well-defined and round edges, while post-treatment, a compromised outer rim integrity was observed in the treatment groups compared to the controls. After the 24-hour exposure period, the nine replicates from each condition were combined and briefly treated with trypsin to create a single-cell suspension. The inventors then employed Fluorescence-Activated Cell Sorting (FACS), using DAPI staining to assess cell viability and efficiently segregate the respective cell populations. Figure 14 illustrates the gating strategy, developed using wild-type (WT) digested cerebral organoids with and without DAPI to identify the live fraction, and RFP-labeled digested cerebral organoids with DAPI to isolate the live RFP-positive fraction. Additionally, 2D GL26-GFP cell suspensions were used to identify the live GFP-positive cancer cell fraction, and the glioma core model was analyzed to obtain the desired subpopulations. This approach allowed the inventors to collect predefined cell groups: viable non-cancerous organoid cells (RFP-positive, DAPI-negative) and viable cancerous cells (GFP-positive, DAPI-negative). Following this, the isolated cell populations were promptly stored at -80°C for subsequent processing. Crucially, these samples were employed for messenger ribonucleic acid (mRNA) extraction and subsequent library preparation, paving the way for next-generation Illumina sequencing. This process facilitates differential mRNA analysis and gene pathway analysis, offering intricate insights into the molecular mechanisms of action, with a particular focus on gene expression regulation post-drug exposure. While the detailed discussion of drug-dependent effects identified in the respective bioinformatic analysis is beyond the scope of this methods paper, the inventors' proof-of-concept experiment demonstrates the potential of the cancer core model of the invention to not only assess toxicity and efficacy but also enable a detailed study of the underlying mechanisms in both cancerous and non-cancerous cells.

### C. Conclusion

The inventors' study introduces innovative 3R-compliant aNSCs-derived models, marking a methodological leap in brain cancer research. These models, particularly the 'assembled', 'cancer core' and 'advanced cancer core' tumor-brain constructs, offer an ethically sound and biologically relevant alternative for early drug discovery and neurotoxicity assessment. By effectively replicating the brain tumor microenvironment, they allow for a more nuanced understanding of tumor dynamics and drug responses.

Aligning with the European 3Rs initiative, the inventors' approach not only addresses ethical concerns but also presents a scalable solution to the high-throughput demands of modern drug discovery.

The potential of these models extends beyond brain cancer, suggesting broader applications in drug discovery and disease modeling. As the inventors refine and validate these systems, they realize that the models will significantly impact the development of safer, more effective therapeutics, reflecting the commitment to advancing scientific methodology while adhering to ethical and responsible research practices.

## Claims

1. A method of generating a tumor assembloid comprising the steps of:
(1) providing a single organoid cell suspension;
(2) providing a single tumor cell suspension;
(3) mixing cells of the single organoid cell suspension with cells of the single tumor cell suspension in a predetermined ratio to obtain an organoid cell-tumor cell mixture;
(4) culturing the organoid cell-tumor cell mixture to obtain the tumor assembloid.

2. The method according to claim 1, **characterized in that** the predetermined ratio of number of cells of the single organoid cell suspension to number of cells of the single tumor cell suspension is in the range of approx. 1:1 to approx. 10,000:1.

3. The method according to claim 1 or 2, **characterized in that** the predetermined ratio of the number of cells of the single organoid cell suspension to the number of cells of the single tumor cell suspension is in a range selected from the group consisting of: approx. 2:1, approx. 4:1, approx. 10:1, approx. 20:1, approx. 100:1, approx. 1,000:1, approx. 2,000:1, approx. 5,000:1, approx. 10,000:1.

4. The method according to any preceding claim, **characterized in that** the tumor cells are derived from a solid tumor, preferably from a tumor of human origin,
further preferably
the tumor cells are selected from the group consisting of: brain cancer cells, breast cancer cells, lung cancer cells, skin cancer cells;
highly preferably
the tumor cells are selected from the group consisting of: glioma cells, breast-to-brain cancer cells, lung-to-brain cancer cells, melanoma-to-brain cancer cells.

5. The method according to any of the preceding claims, **characterized in that** the organoid cells are derived from a cerebral organoid,
preferably
the cerebral organoid was obtained from cells of the group consisting of: adult neural stem cells (aNSCs), embryonic stem cells (eSC), and induced pluripotent stem cells (iPSC),
further preferably
the cerebral organoid was grown for at least 1 month.

6. The method according to any of the preceding claims, **characterized in that** it comprises the following further steps:
(2') providing a single immune cell suspension;
(3') mixing cells of the single organoid cell suspension with cells of the single tumor cell suspension and with cells of the single immune cell suspension in a predetermined ratio to obtain an organoid cell-tumor cell-immune cell mixture, and
(4') culturing the organoid cell-tumor cell-immune cell mixture to obtain an 'advanced' tumor assembloid.

7. The method according to claim 6, **characterized in that** the predetermined ratio of the number of cells of the single organoid cell suspension to the number of cells of the single tumor cell suspension and to the number of cells of the single immune cell suspension is in a range of approx. 2:1:1 to approx. 100:1:100.

8. The method according to claim 6 or 7, **characterized in that** the immune cells are cells selected from the group consisting of: microglia cells, monocytes, macrophages, T lymphocytes, B lymphocytes, and dendritic cells.

9. A tumor assembloid obtainable by the method according to any of claims 1-8.

10. A method of generating a tumor spheroid assembloid comprising the steps of:
(1) providing a single organoid cell suspension;
(2) providing preformed tumor spheroids;
(3) mixing cells of the single organoid cell suspension with the preformed tumor spheroids in a predetermined ratio to obtain an organoid cell-tumor spheroid mixture;
(4) culturing the organoid cell-tumor spheroid mixture to obtain the tumor spheroid assembloid.

11. The method according to claim 10, **characterized in that** the predetermined ratio of the number of cells of the single organoid cell suspension to the number of tumor spheroids is approx. 10,000 to approx. 500,000 cells to approx. a single preformed tumor spheroid.

12. The method according to claim 10 or 11, **characterized in that** the single preformed tumor spheroid comprises approx. 1,000 to approx. 100,000 tumor cells.

13. The method according to any of claims 10 to 11, **characterized in that** it comprises the following further steps:
(2') providing a single immune cell suspension;
(3') mixing cells of the single organoid cell suspension with the preformed tumor spheroids and with cells of the single immune cell suspension in a predetermined ratio to obtain an organoid cell-tumor spheroid-immune cell mixture, and
(4') culturing the organoid cell-tumor spheroid-immune cell mixture to obtain an 'advanced' tumor spheroid assembloid.

14. The method according to claim 20, **characterized in that** the predetermined ratio of the number of cells of the single organoid cell suspension to the number of pre-formed tumor spheroids and to the number of cells of the single immune cell suspension is approx. 10,000 to approx. 500,000 cells of the single organoid cell suspension to approx. a single preformed tumor spheroid and approx. 10,000 to approx. 500,000 cells of the single immune cell suspension.

15. A tumor spheroid assembloid obtainable by the method according to claim 10 to 14.
